(19) Europäisches Patentamt
European Patent Office
Office européen des brevets

(11) Publication number: **0 559 048 A1**

(12) # EUROPEAN PATENT APPLICATION

(21) Application number: **93102790.8**

(22) Date of filing: **23.02.93**

(51) Int. Cl.$^5$: **G03C 8/10**, C07D 413/12

(30) Priority: **24.02.92 JP 72114/92**

(43) Date of publication of application: **08.09.93 Bulletin 93/36**

(84) Designated Contracting States: **DE FR GB**

(71) Applicant: **FUJI PHOTO FILM CO., LTD.**
**210 Nakanuma Minami Ashigara-shi**
**Kanagawa(JP)**

(72) Inventor: **Uchida, Osamu, c/o Fuji Photo Film Co., Ltd.**
**210, Nakanuma**
**Minami Ashigara-shi, Kanagawa(JP)**
Inventor: **Matsuda, Naoto, c/o Fuji Photo Film Co., Ltd.**
**210, Nakanuma**
**Minami Ashigara-shi, Kanagawa(JP)**
Inventor: **Nakamura, Koki, c/o Fuji Photo Film Co., Ltd.**
**210, Nakanuma**
**Minami Ashigara-shi, Kanagawa(JP)**
Inventor: **Hirano, Katsumi, c/o Fuji Photo Film Co., Ltd.**
**210, Nakanuma**
**Minami Ashigara-shi, Kanagawa(JP)**

(74) Representative: **Patentanwälte Grünecker, Kinkeldey, Stockmair & Partner**
**Maximilianstrasse 58**
**D-80538 München (DE)**

(54) **Diffusion transfer type silver halide photographic light-sensitive material.**

(57) A diffusion transfer type silver halide photographic light-sensitive material contains a dye-providing compound having a dye portion temporarily shifted to a shorter wavelength. The light-sensitive material contains the dye-providing compound represented by the following Formula (I):

REDOX-NuP-E-G-Dye' (I)

wherein G represents an auxochrome of a dye; Dye' represents a group of atoms which remains after the auxochrome is removed from the dye; -G-Dye' represents a group of atoms which is converted to a diffusible dye by cleavage of the bond between G and E; REDOX represents an oxidative group or a reductive group which allows the bond between REDOX-NuP to be cleaved by an oxidation-reduction reaction; NuP represents a nucleophilic group which is released by a bond cleavage reaction between REDOX and NuP triggered by the oxidation-reduction reaction; E represents an electrophilic group which will react with the nucleophilic group NuP and cause the bond between G and E to be cleaved; and REDOX-NuP-E provides the compound represented by Formula (I) with a diffusibility.

EP 0 559 048 A1

## FIELD OF THE INVENTION

The present invention relates to a diffusion transfer type silver halide photographic light-sensitive material. More specifically, the invention relates to a diffusion transfer type silver halide photographic light-sensitive material containing a dye-providing compound whose dye moiety has a chromophoric absorption temporarily shifted to shorter wavelengths during exposure, but converted to the desired absorption through a novel mechanism upon processing.

## BACKGROUND OF THE INVENTION

Diffusion transfer type silver halide photographic light-sensitive materials generally contain dye-providing compounds. The dye-providing compounds are those containing a dye moiety having an absorption in the visible light region and have a mechanism by which the mobility of the dye-providing compounds or the mobility of the dye moiety is changed corresponding to or reversely corresponding to the development of silver halide. Simultaneous with or after the development the mobile dye-providing compounds or the mobile dye moieties are transferred to an image-receiving layer to form an image. In view of this principle, a dye or a dye-providing compound is preferably allowed to be present in the vicinity of silver halide in the light-sensitive material and this means that the dye or dye-providing compound is preferably allowed to be present in the same layer as a silver halide emulsion in designing the light-sensitive material.

However, this causes a problem recognized in the art. Incorporating a dye or a dye-providing compound and a silver halide emulsion in the same layer allows the absorption wavelength of the dye to overlap with that of the spectrally sensitized silver halide grains and therefore the dye, acting as a filter, reduces the quantity of light striking the silver halide grains, which in turn lowers the sensitivity of the light-sensitive material (hereinafter referred to as a filter effect).

This filter effect problem makes it substantially impossible to allow a dye or a dye-providing compound and a silver halide emulsion to be present in the same layer when a diffusion transfer type silver halide color light-sensitive material is to be used for a photographing application. Instead, each dye or dye-providing compound is located in a separate layer beneath the appropriately sensitized emulsion layer (yellow dye or dye-providing compound below blue-sensitized emulsion, and so on). This configuration is necessary to avoid speed losses from light absorption which would result if the dyes or dye-providing compounds were coated in the emulsion layers. See T.H. James, The Theory of the Photographic Process 367 (4th ed. 1977).

This layer differentiation can prevent speed losses but is hardly ideal compared with the principle of the design mentioned previously and generates a new problem. An explanation will be given below citing an example of a light-sensitive material which is constructed with an immobilized compound which is reducible so as to release a diffusible dye.

In a light-sensitive material in which a so-called positive-working compound is used as a dye-providing compound as disclosed in, for example, U.S. Patent 4,783,396 image formation is effected by the competition between a redox reaction of a positive-working dye-providing compound with a reducing agent and a silver developing reaction of the reducing agent with the exposed silver halide. In other words, the minimum densities occur in areas where all of the reducing agent is consumed by the silver developing reaction and the reaction of the positive-working compound with the reducing agent does not take place so that no dye is released. Maximum densities occur in areas where the silver developing reaction does not take place, the reducing agent remains, and the positive-working compound is reduced to release the dye.

Obtaining an image having a good S/N ratio (Dmax/Fog Density ratio) with such a light-sensitive material requires a system in which the reaction of the exposed silver halide with the reducing agent is favored over the reaction of the reducing agent with the positive-working compound, and the combined use of an electron transfer agent is effective for this purpose. The electron transfer agent reacts with exposed silver halide and is converted to an electron transfer agent oxidation material. The electron transfer agent oxidation material in turn oxidizes the reducing agent present in its vicinity. In this case, as the diffusing distance of the electron transfer agent oxidation material decreases, the relationship between silver development and consumption of the reducing agent improves, giving an image with a good S/N ratio. However, the above mentioned layer differentiation requires an increase in the diffusion distance of the electron transfer agent oxidation material. Further, an increase in the diffusion distance of the electron transfer agent oxidation material in turn increases diffusion of the electron transfer agent oxidation material to other light-sensitive layers, which suppresses color development in the other layers and therefore deteriorates the color reproducibility of the image.

Temporarily shifting the chromophoric absorption of a dye or dye-providing compound to shorter wavelengths during exposure has been proposed as a means to minimize the filter effect and eliminate the need for the above-mentioned layer differentiation. For example, shifting the absorption wavelength of a magenta dye in a light-sensitive material to a shorter wavelength in the yellow region would reduce the overlap with the absorption wavelength of a silver halide emulsion spectrally sensitized to green light and prevent speed losses due to the filter effect. Incorporating a dye or dye-providing compound and a silver halide emulsion in the same layer would become possible, which would prevent reduction of image quality originating from the above-mentioned practice of coating the dye or dye-providing compound in a separate layer beneath the corresponding photographic emulsion. Accordingly, it can be said that temporarily shifting the chromophoric absorption of a dye or dye-providing compound to a shorter wavelength is very important and effective for designing a diffusion transfer type silver halide color light-sensitive material. See The Theory of the Photographic Process, supra, at 368.

The temporary shorter wavelength shift has been extensively investigated and reported. The main subject of investigation has been the compatibility of the storage stability of the temporary shorter wavelength shift effect with rapid recovery of dye hue in processing. Certain azo dyes are available as compounds which have been used effectively as image-forming dyes in diffusion transfer type silver halide color light-sensitive materials. In general, an auxochrome is protected by an acyl group as the means for subjecting the azo dye to the temporary shorter wavelength shift as disclosed in JP-A-50-26541 (the term "JP-A" as used herein means an unexamined published Japanese patent application),and U.S. Patent 3,999,991. In this method, the auxochrome is regenerated by a hydrolysis reaction with an alkali supplied during processing to allow the hue to be recovered. In general, however, compounds in which the hue is rapidly recovered during processing are susceptible as well to undergoing hydrolysis during storage of the light-sensitive material and have problems with respect to the stability of the temporary shorter wavelength-shift effect. There are available as means for solving this problem associated with the acylation-alkali hydrolysis, for example, a method in which an intermolecular nucleophilic reaction of an adjacent group is utilized, as disclosed in JP-A-55-53329, and a method using a compound having a precursor which can be converted to a group capable of causing an intermolecular nucleophilic reaction by the action of an alkali, as disclosed in JP-A-55-53330 and U.S. Patent 4,310,612. However, it can not be said that these methods offer a complete solution for the storage stability/rapid hue recovery problem. It would be preferable to have a system which is storage-stable and yet allows rapid hue recovery during processing merely based on the pH change which occurs during processing.

What is considered to be a better approach to making stability in the temporary shorter wavelength shift compatible with rapid hue recovery is to design the system so that the hue recovery takes place at the same time as the dye-releasing reaction from a dye-providing material or so that the dye-releasing reaction acts as a trigger for the hue recovery. In other words, the approach is to use a temporary shorter wavelength-shift dye-providing material for which an oxidation-reduction reaction occurring during processing acts as a switch to activate hue recovery. It is expected that this method would make it possible to use a dye auxochrome protective group having improved storage properties in comparison to the dye auxochrome protective groups which may be used in the above-described alkali hydrolysis method.

For example, it can be said that a reducible dye-providing material in which a dye and a positive-working compound are combined via an auxochrome is the most reasonable molecule design in terms of achieving rapid hue recover during processing since the hue is recovered at the same time as the reducible dye-providing material is reduced by the reaction with a reducing agent to allow the dye to be released. The usefulness of such a compound has not so far been described in detail but compounds of this type are described in British Patent 1,596,828 and U.S. Patent 4,783,396. In particular, it can be said that the use of a dye-providing compound which is subjected to a temporary shorter wavelength shift as described in U.S. Patent 4,783,396 allows the hue to be recovered at the same time as the dye is released by an electron transfer reaction and therefore that it has excellent characteristics in terms of hue recovery speed. However, it is difficult to synthesize some of the dyes.

With regard to the reducible dye-providing compounds described in British Patent 1,596,828, the reactivity of these compounds toward a reducing agent has a close relationship with the deprotection speed by an intermolecular nucleophilic addition reaction. The resultant increase in reducibility leads to a lack of nucleophilicity which decreases the hue recovery speed, so that it has been difficult to adjust the redox activity and the deprotection speed.

In sum, there are problems associated with the known dyes and dye-providing compounds whose chromophoric absorption has been temporarily shifted to shorter wavelengths. Many of the known dyes and dye-providing compounds do not achieve a satisfactory balance between storage stability and hue recovery during processing. While certain dyes and dye-providing compounds do provide a satisfactory balance in

this regard, they are difficult to synthesize.

## SUMMARY OF THE INVENTION

A first object of the present invention is to provide a diffusion transfer type silver halide color photographic material which includes a dye-providing comopund whose dye moiety has a chromophoric absorption temporarily shifted to shorter wavelengths during exposure, but convented to the desired absorption through a novel mechanism upon processing and which has good sensitivity.

A second object of the present invention is to provide a diffusion transfer type silver halide color photographic material in which the above-described dye-providing compounds are used to reduce the filter effect and resultant speed losses which may occur when non-shifted dye-providing compounds are incorporated in the same layer as the corresponding photographic emulsion.

Extensive investigations were made by the present inventors based on the judgment that to provide a shifted dye-providing compound having a good balance between storage stability and rapid hue recovery upon processing, the best approach would be to design a molecule in which the hue recovery reaction is triggered by an oxidation-reduction reaction. As a result of their investigations, the inventors have found that a nucleophilic group is generated when the oxidation-reduction reaction is used as a trigger. Further, the inventors have found that the intermolecular nucleophilic reaction of the nucleophilic group can be utilized to achieve rapid hue recovery upon processing of the shifted dye-providing compound.

That is, the objects of the present invention have been achieved by a diffusion transfer type silver halide photographic light-sensitive material containing a dye-providing compound represented by the following Formula (I):

REDOX-NuP-E-G-Dye'     (I)

wherein G represents an auxochrome of a dye; Dye' represents a group of atoms which remains after the auxochrome is removed from the dye; -G-Dye' represents a group of atoms which is converted to a diffusible dye by cleavage of the bond between G and E; REDOX represents an oxidative group or a reductive group which allows the bond between REDOX and NuP to be cleaved by an oxidation-reduction reaction; NuP represents a nucleophilic group which is released by a bond cleavage reaction between REDOX and NuP triggered by the oxidation-reduction reaction; and E represents an electrophilic group which will react with the nucleophilic group NuP and cause the bond between G and E to be cleaved.

## DETAILED DESCRIPTION OF THE INVENTION

Formula (I) is described in detail below.

In Formula (I), REDOX represents a group capable of releasing -Nu-P-E-G-Dye' by a reaction which is triggered by an oxidation-reduction reaction.

When the compound represented by Formula (I) are reducing ones, preferred examples of REDOX include the ortho-sulfonamidophenols described in JP-A-59-60434 and the hydrazine derivatives described in U.S. Patent 3,844,785.

When the compound represented by Formula (I) is an oxidative compound, preferred examples of REDOX include a group in which a cleavage reaction of a nitrogen-oxygen single bond is utilized, as described in U.S. Patent 4,783,396, a group in which a cleavage reaction of a nitrogen-sulfur single bond is utilized, as described in JP-A-62-244048, the groups described in JP-A-61-88257, and the groups described in JP-A-3-40498. Further, there can be used a group in which an intermolecular nucleophilic reaction is utilized, as described in British Patent 1,596,828.

In Formula (I), NuP represents a nucleophilic group precursor which expresses a nucleophilicity after the cleavage between REDOX and NuP. The mechanism by which a nucleophilicity is expressed may be, for example, an electron transfer reaction and a decarboxylation reaction or it may include a hydrolysis reaction or it may be a nucleophilic substaitution reaction of a nucleophilic agent supplied externally, for example, hydroxylamine. The number of examples of the nucleophilic group precursor represented by NuP are considered limitless when considering the combinations with REDOX. Preferred examples of nucleophilic groups are described below.

Phenols (phenols which may be substituted, for example, with an alkyl group, an alkoxy group, an acyl group, a halogen atom, an alkylsulfonyl group, an arylsulfonyl group, a cyano group or a nitro group), for example, phenol, 1-naphthol, para-methoxyphenol, para-acetylaminophenol, meta-chlorophenol, para-cyanophenol, and para-nitrophenol;

Thiophenols (thiophenols which may be substituted, for exmaple, with an alkyl group, an alkoxy group, an acyl group, a halogen atom, an alkylsulfonyl group, an arylsulfonyl group, a cyano group or a nitro group), for example, thiophenol, para-cyanothiophenol, para-methoxythiophenol, meta-methoxyethoxythiophenol, and para-chlorothiophenol;

Sulfonylamino groups (sulfonylamino groups which may be substituted, for example, methanesulfonylamino, trifluoromethanesulfonylamino, ethanesulfonylamino, benzenesulfonylamino, and p-nitrobenzenesulfonylamino; and

Hydroxamic acids (hydroxamic acids which may be substituted, for example, with an alkyl group or an aryl group), for example, N-methylhydroxamic acid, N-phenylhydroxamic acid, N-ethylhydroxamic acid, N-para-cyanophenylhydroxamic acid, and N-isopropylhydroxamic acid.

NuP is a divalent group such as the above-described phenols, thiophenols and hydroxamic acids. When NuP is one of the phenols, -REDOX is linked to the oxygen atom of the OH group and -E-G-Dye' is linked to the ortho position. When NuP is one of the thiophenols, -REDOX is linked to the sulfur atom of the SH group and -E-G-Dye' is linked to the ortho position. When NuP is one of the hydroxaminc acids, -REDOX is linked to the oxygen atom constituting the hydroxaminc acid and -E-G-Dye' is linked to the carbonyl group. Group, -E-G-Dye', may be linked to the ortho position of the phenols, the ortho position of the thiophenols and the carbonyl group of the hydroxamic acids. If these ortho positions and the carbonyl group have a substituent, -E-G-Dye' mayb e linked to the substituent. Examples of the latter linkages include a linkage of -E-G-Dye' through an alkylene group.

Of these nucleophilic groups, the substituted phenols are most preferred because they are readily synthesized.

G represents an auxochrome of a diffusible dye and may be either a single atom or a group of atoms. Preferably, G represents an oxygen atom or an amino group, more preferably an oxygen atom.

E represents a divalent atomic group which acts as a protective group for the auxochrome and which reacts with NuP having become the nucleophilic group to regenerate G. Preferred examples of E include -CO-, -OCO-, $-NR^3CO-$, -SCO-, -CS-, -OCS-, $-NR^3CS-$, -SCS-, -SO-, and $-SO_2-$, wherein $R^3$ represents a hydrogen atom, an alkyl group (which may be substituted), or an aryl group (which may be substituted). $R^3$ may link with NuP to form a ring.

The shifted dye-providing compounds of the present invention release a nucleophilic group after an oxidation-reduction reaction and accordingly, a preferred result in releasing a nucleophilic group (NuP) is easy to obtain when the compound of Formula (I) is a reducible dye-providing compound.

When the shifted dye-providing compound represented by Formula (I) is a reducible dye-providing compound, the compounds of Formula (I) are preferably represented by Formula (II) below. The compounds of Formula (II) have excellent reactivity with reducing agents and excellent storage stability:

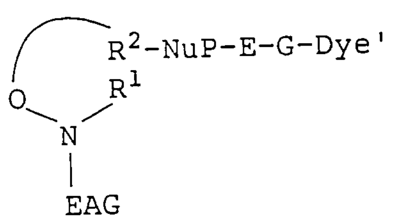

(II)

In Formula (II), EAG represents a reducible electron-accepting group; $R^2$ represents a group of atoms which cleaves the bond between $R^2$-NuP when triggered by the cleavage of a nitrogen-oxygen single bond; $R^1$ represents a substituent other than a hydrogen atom; $R^1$ and $R^2$ may be combined with each other to form a ring; and NuP, E, G and Dye' have the same meanings as in Formula (I).

In Formula (II), EAG represents an electron-accepting group. Examples of EAG include an aryl group substituted with at least one electron attractive group (for example, 4-nitrophenyl, 2-nitro-4-N-methyl-N-octadecylsulfamoylphenyl, 2-N,N-dimethylsulfamoyl-4-nitrophenyl, 2-cyano-4-octadecylsulfonyl-phenyl, 2,4-dinitrophenyl, 2,4,6-tricyanophenyl, 2-nitro-4-N-methyl-N-octadecylcarbamoylphenyl, 2-nitro-5-octylthiophenyl, 2,4-dimethanesulfonylphenyl, 3,5-dinitrophenyl, 2-chloro-4-nitro-5-methylphenyl, 2-nitro-3,5-dimethyl-4-tetradecylsulfonylphenyl, 2,4-dinitro-naphthyl, 2-ethylcarbamoyl-4-nitrophenyl, 2,4-bis-dodecylsulfonyl-5-trifluoromethylphenyl, 2,3,4,5,6-pentafluorophenyl, 2-acetyl-4-nitrophenyl, 2,4-diacetylphenyl, and 2-nitro-4-trifluoromethylphenyl); a substituted or unsubstituted heterocyclic ring (for example, 2-pyridyl, 2-pyradyl, 5-nitro-pyridyl, 5-N-hexadecylcarbamoyl-2-pyridyl, 4-pyridyl, 3,5-dicyano-2-pyridyl, 5-dodecylsulfonyl-2-pyridyl, 5-cyano-2-pyradyl, 4-nitrothiophene-2-yl, 5-nitro-1,2-dimethylimidazole-4-yl, 3,5-diacetyl-2-pyridyl, and 1-dodecyl-5-carbamoyl-pyridinium-2-yl); a substituted or unsubstituted quinone (for example, 4-benzoquinone-2-yl, 3,5,6-trimethyl-1,4-benzoquinone-2-yl, 3-methyl-1,4-naphthoquinone-2-yl, 3,6-dimethyl-5-hexadecylthio-1,4-benzoquinone-2-yl, and 5-pentadecyl-1,2-benzoquinone-4-yl); vinylogs of the groups mentioned above, as well as nitroalkane and an α-diketo compounds.

$R^2$ may be any group of atoms as long as it releases NuP when triggered by a nitrogen-oxygen single bond cleavage reaction. Preferably, $R^2$ represents a group of atoms forming a hetrocyclic ring containing a nitrogen-oxygen single bond together with $R^1$. The hetrocyclic rings shown below are preferred examples of $R^2$, wherein the mark ** represents a site to bond to EAG and the mark *** represents a site to bond to NuP.

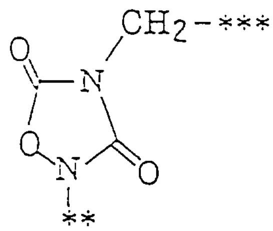
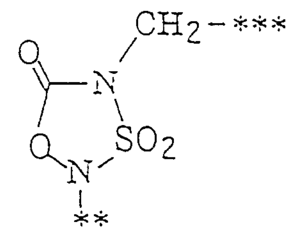
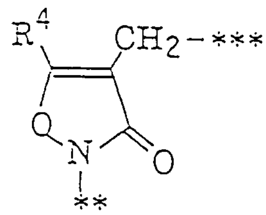
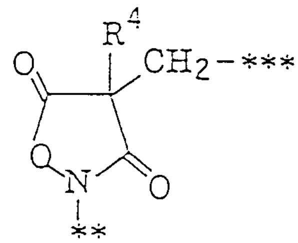
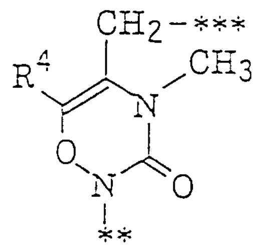
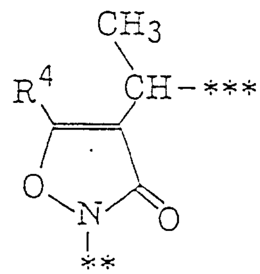
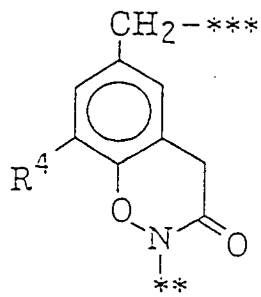
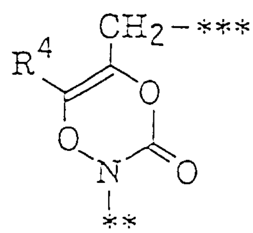
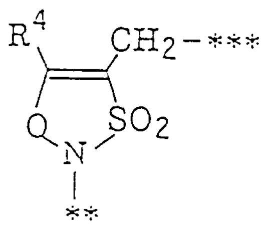
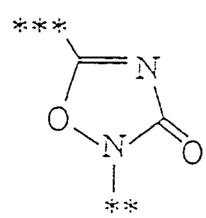

In the above ring structures, $R^4$ represents hydrogen; an alkyl group having preferably 25 or less carbon atoms which may be substituted, for exmaple, with an aryl, alkoxy, aryloxy, acyl, alkylthio, arylthio, carboxyl, sulfo or hydroxyl group or a halogen atom, with preferred examples of alkyl groups including a methyl, ethyl, t-butyl, octadecyl, phenethyl and carboxymethyl groups; an aryl group having preferably 30 or less carbons which may be substituted, for example, with an alkyl, alkoxy, acyl, alkylthio, carboxyl, sulfo,

hydroxyl, nitro, alkylsulfonyl or acylamino group, with preferred examples of aryl groups including a phenyl, 3-nitrophenyl, 4-methoxyphenyl, 4-acetylaminophenyl, 4-methanesulfonylphenyl and 4-tetradecylphenyl groups; and a heterocyclic group of a 5- or 6- meembered ring containing O, N and/or S, with preferred examples of heterocyclic groups including a 2-pyridyl and 2-furyl groups.

Specific examples of compounds for use in the present invention are given below. The present invention is not limited to these compounds.

1

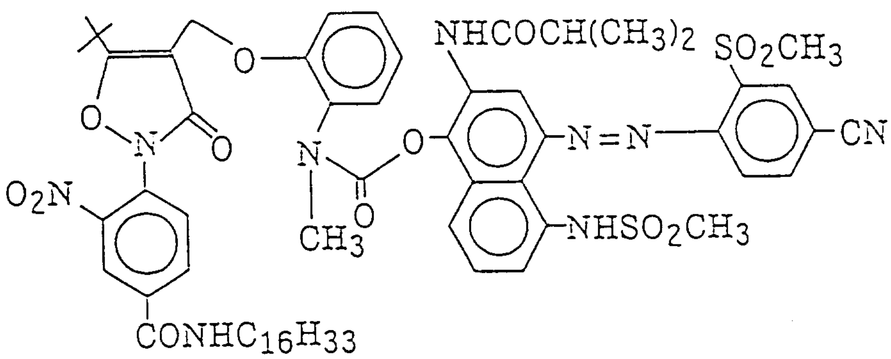

2

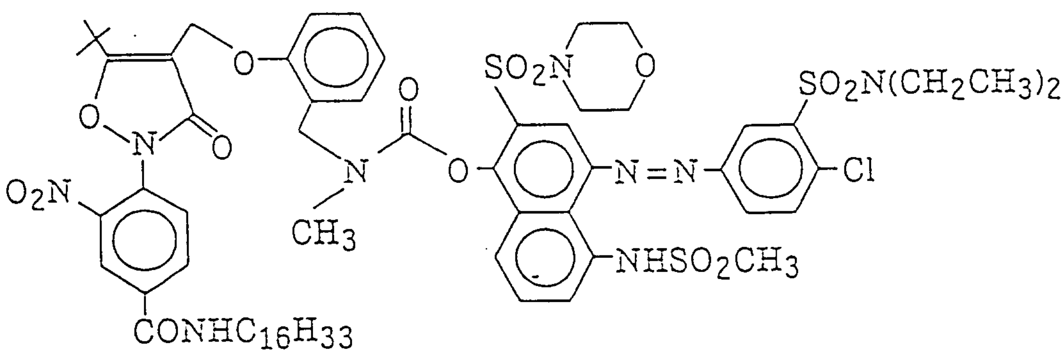

3

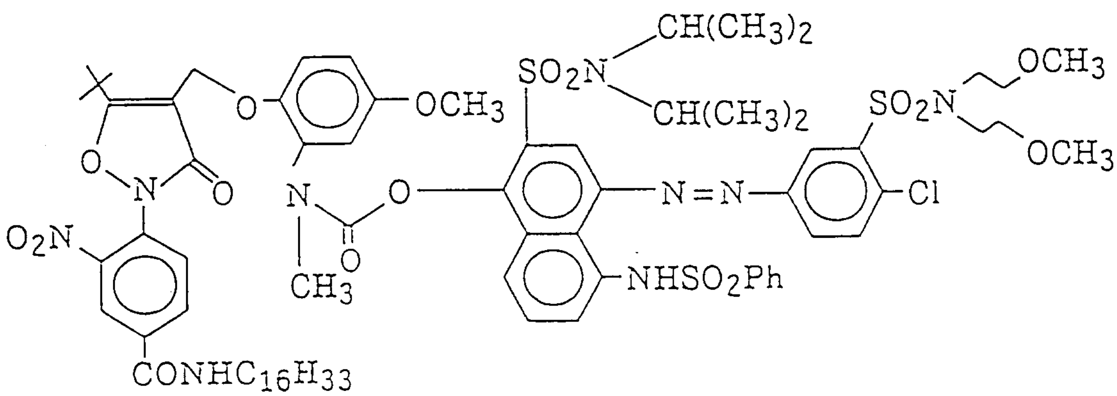

4

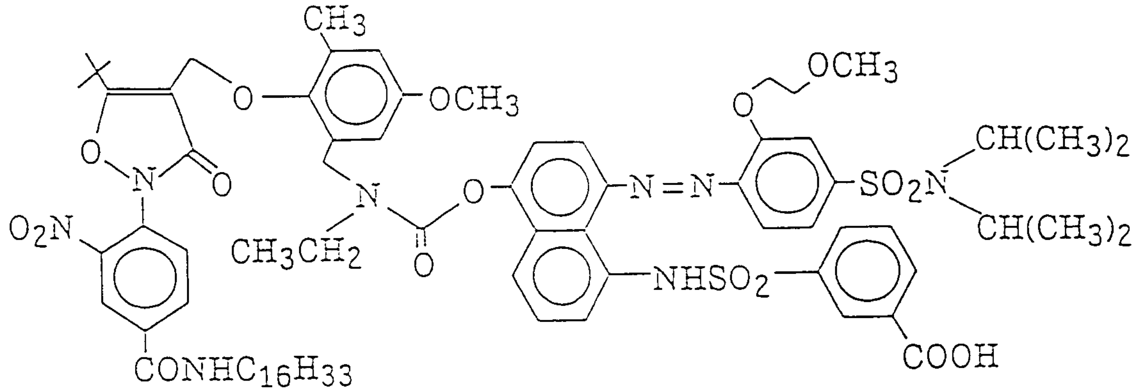

5

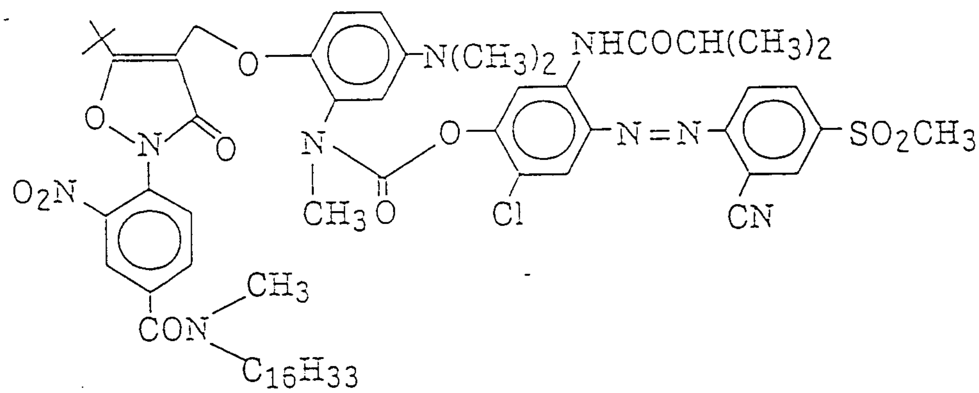

6

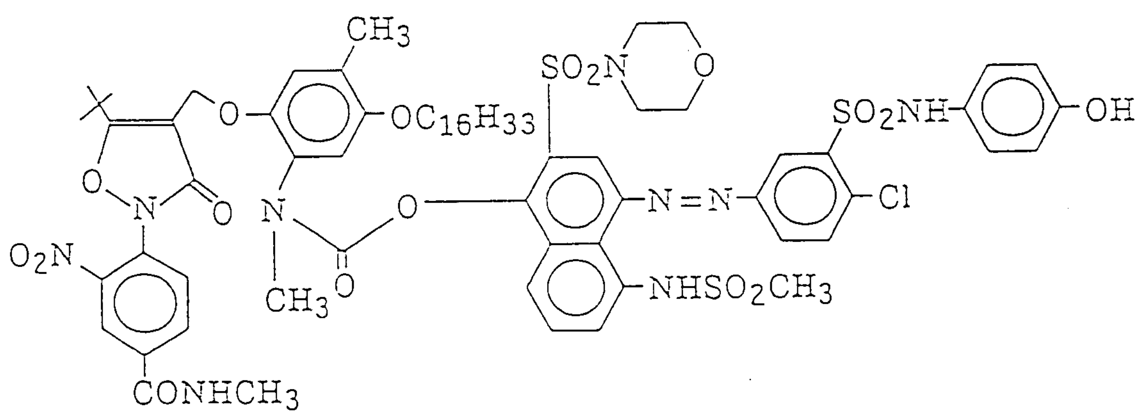

7

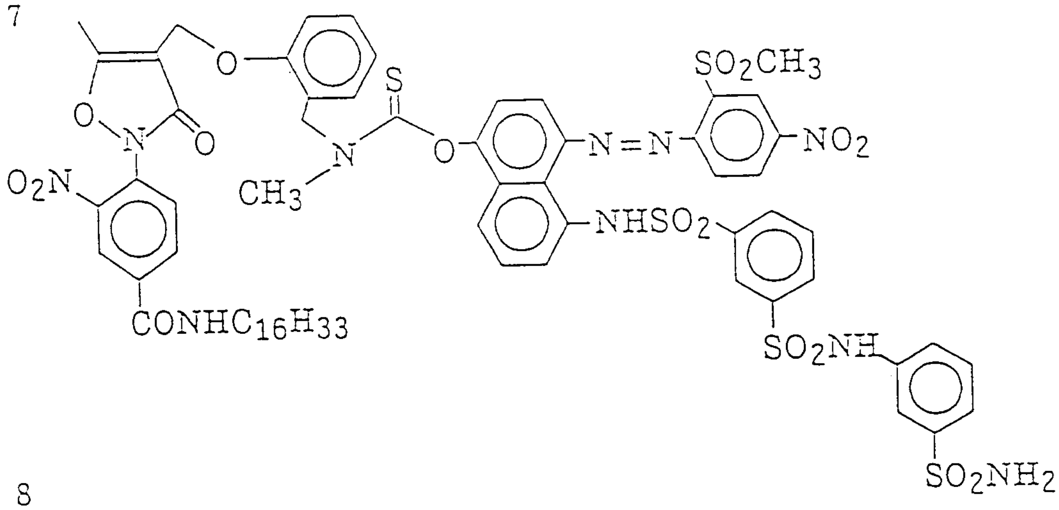

8

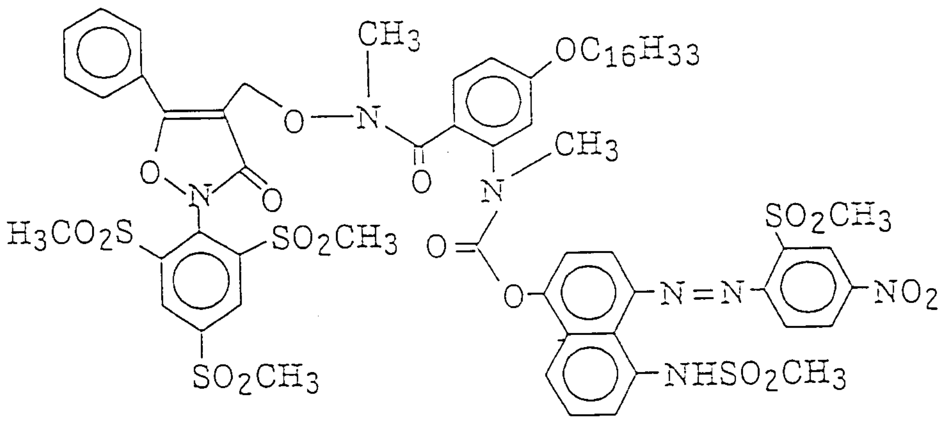

9

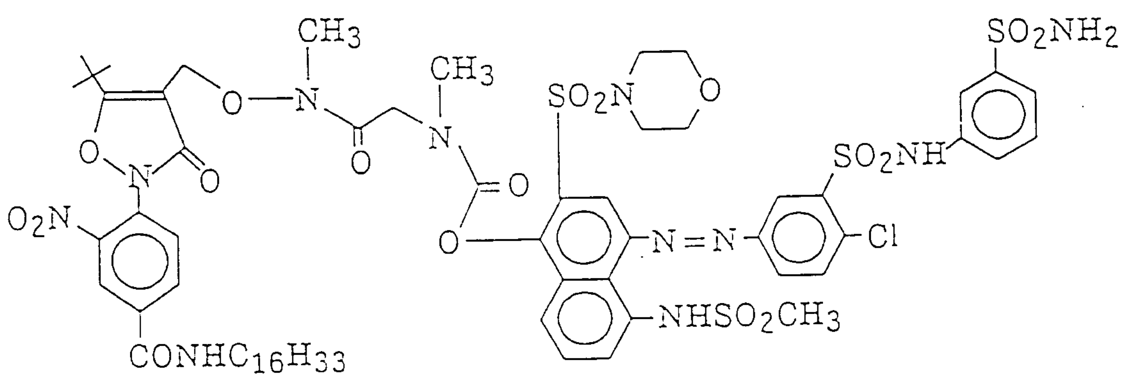

10

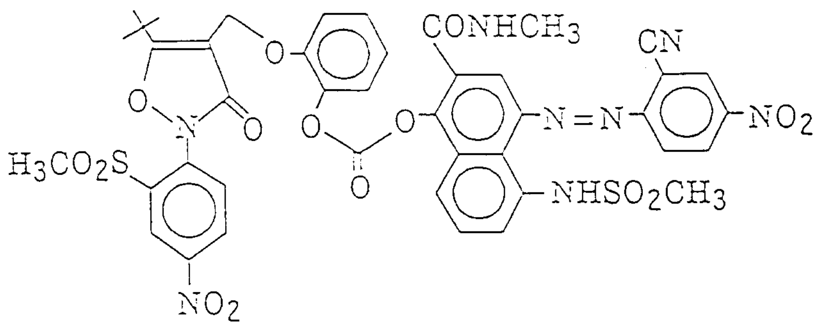

11

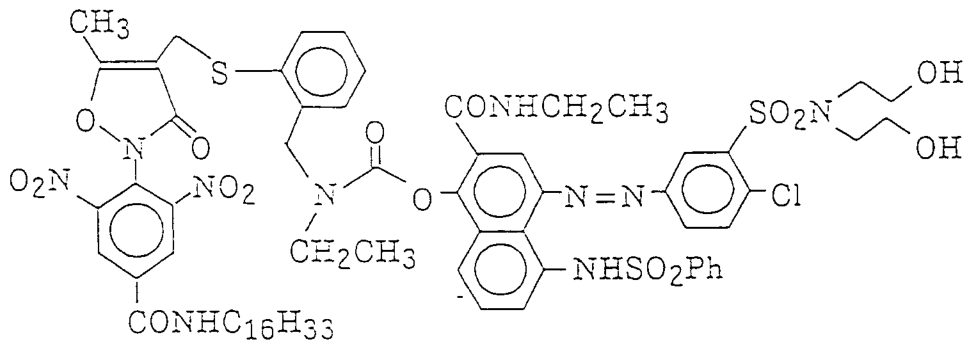

12

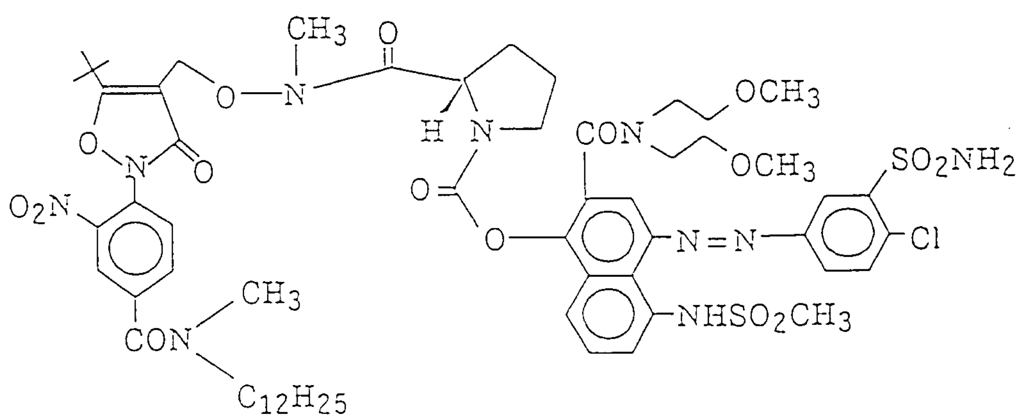

13

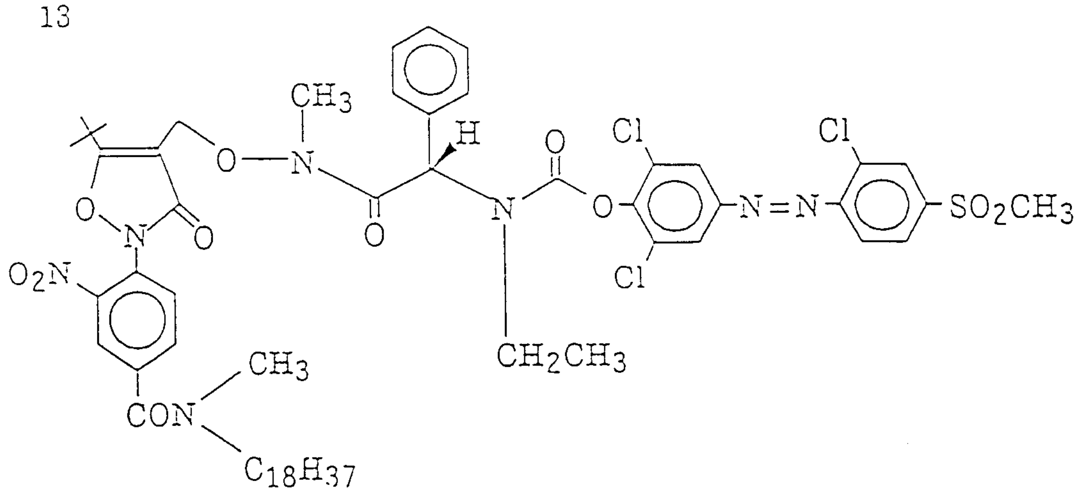

14

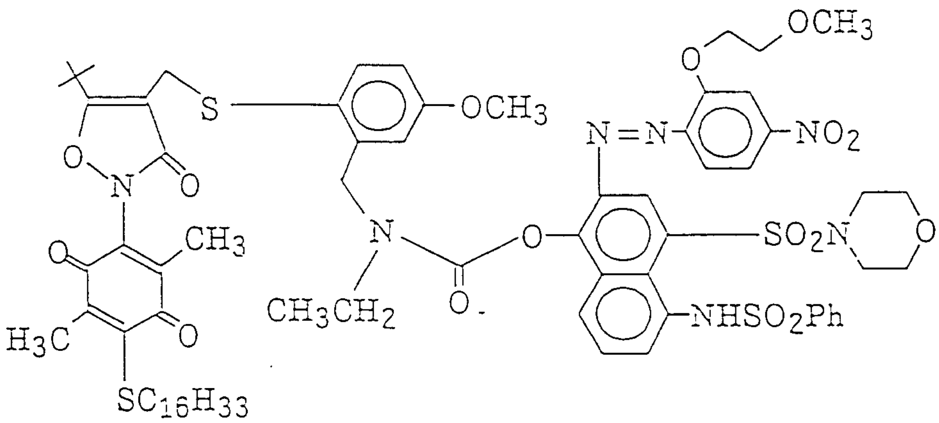

15

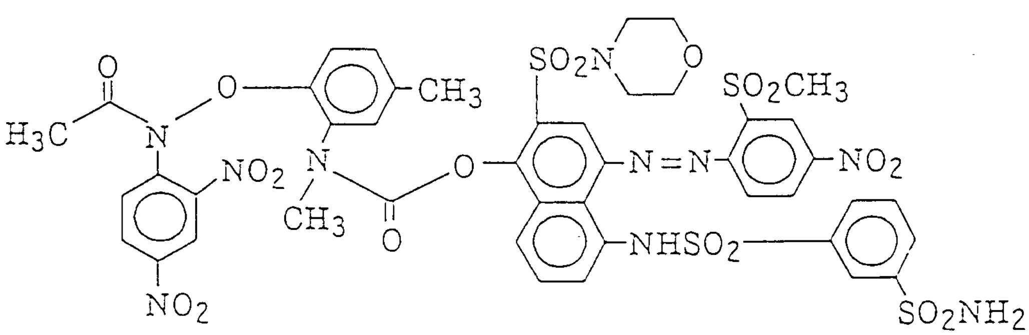

16

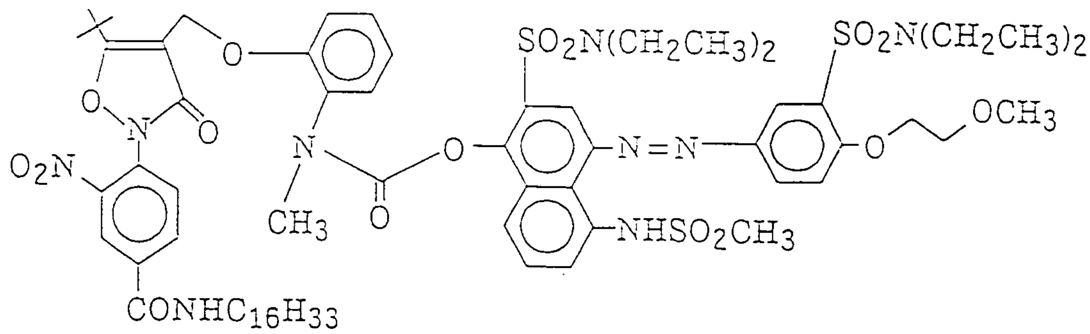

17

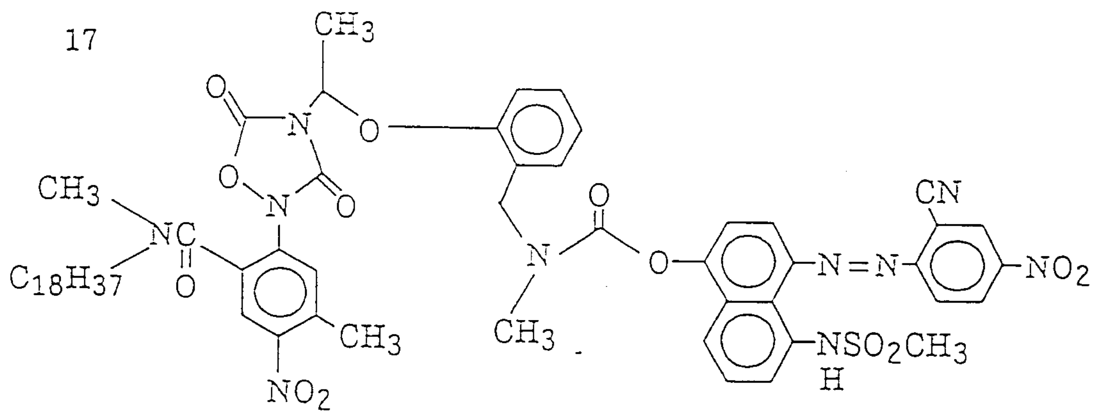

18

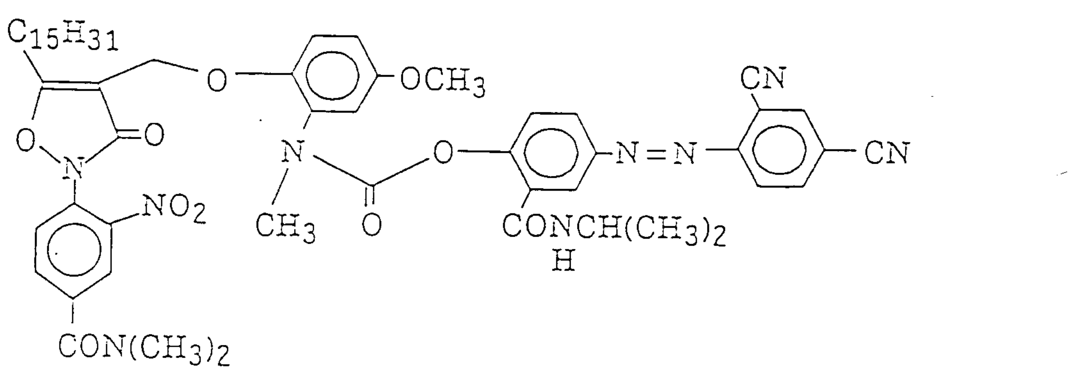

19

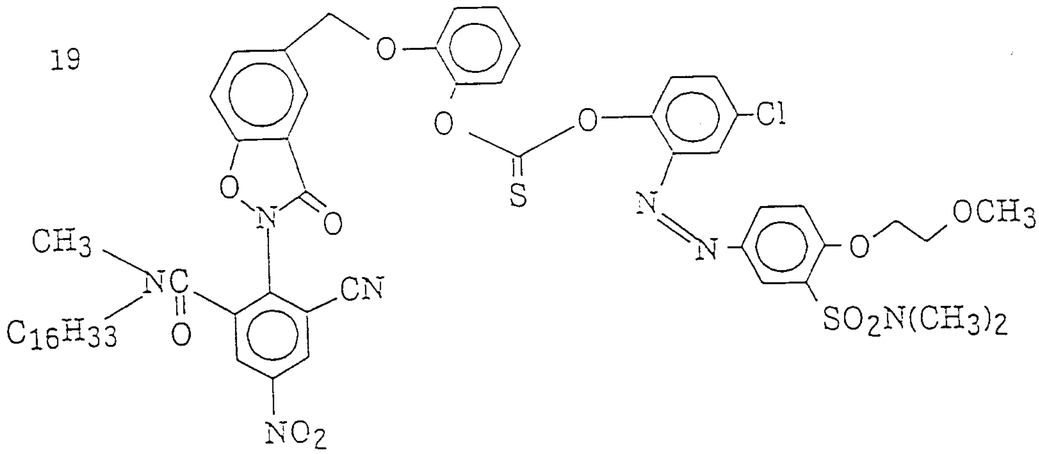

20

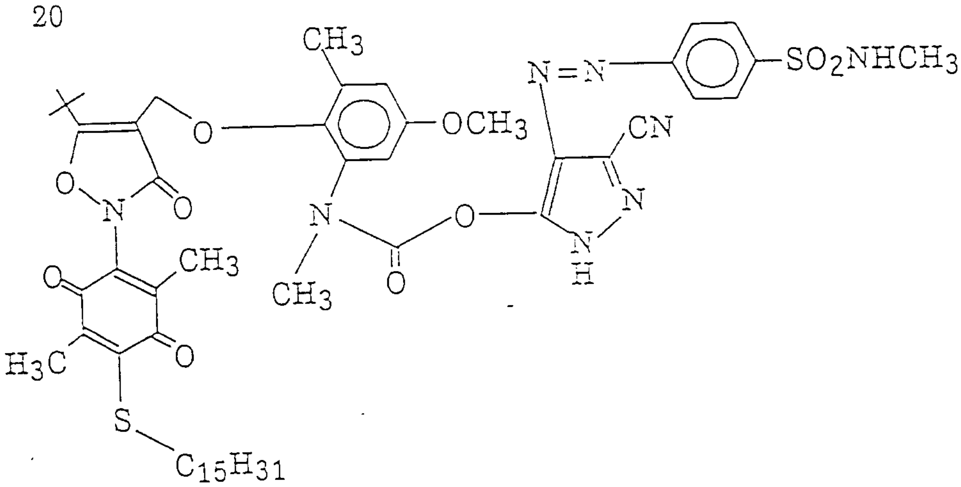

21

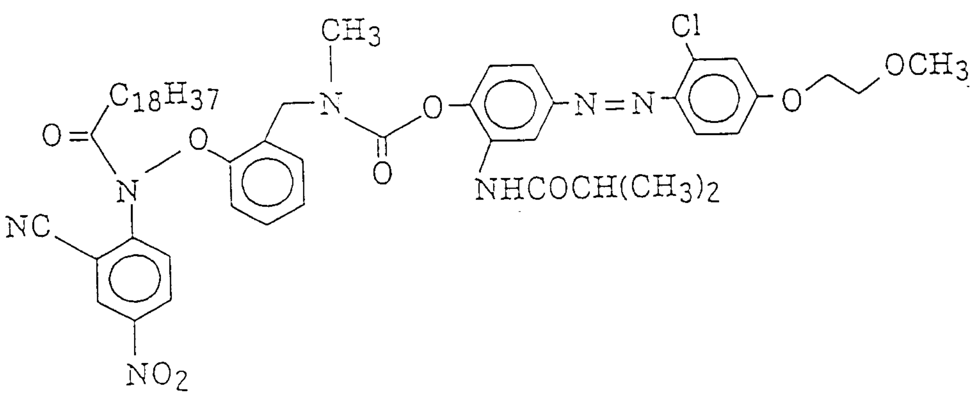

22

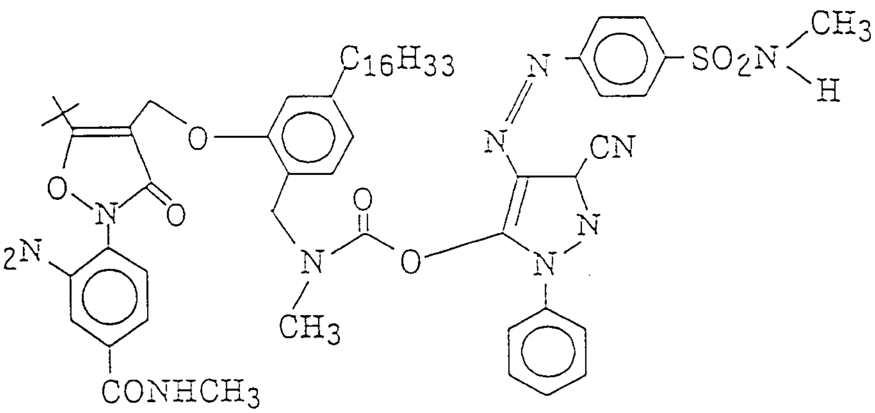

23

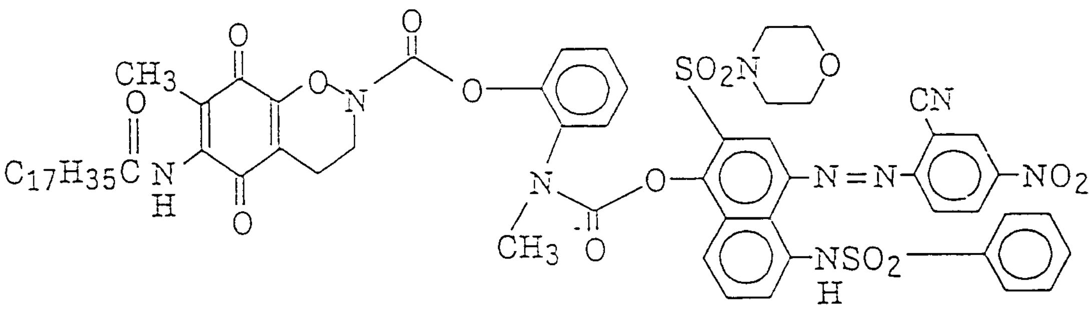

24

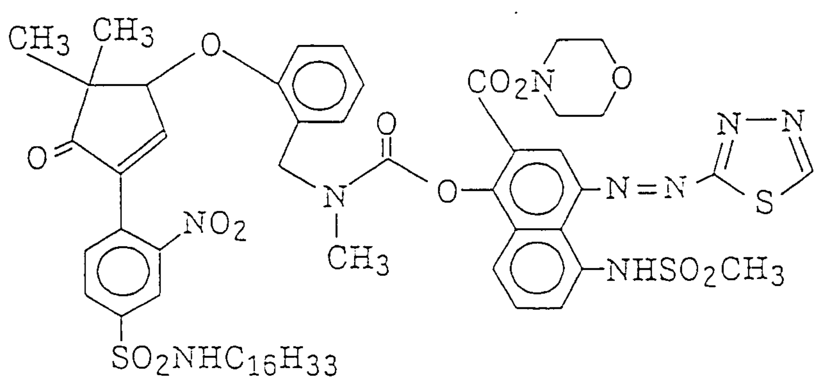

25

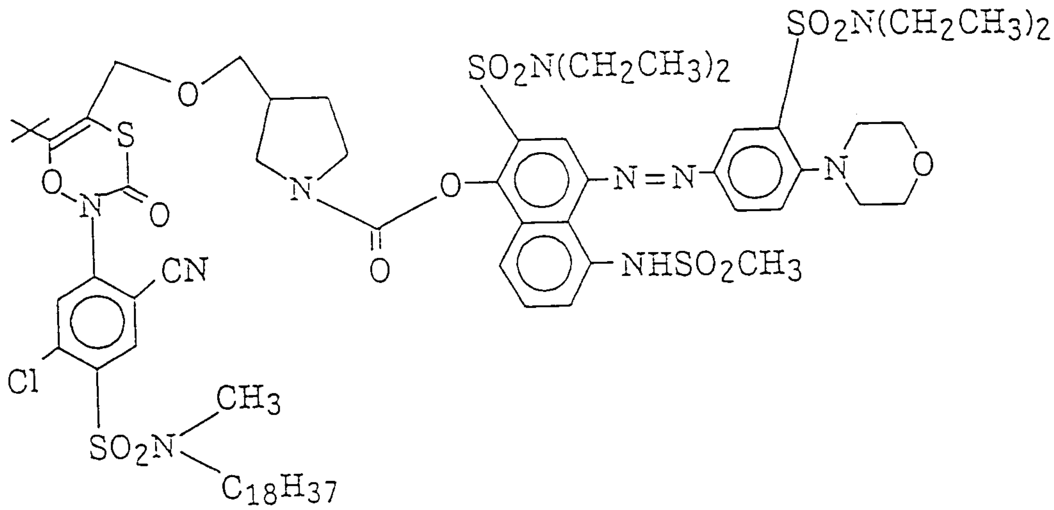

26

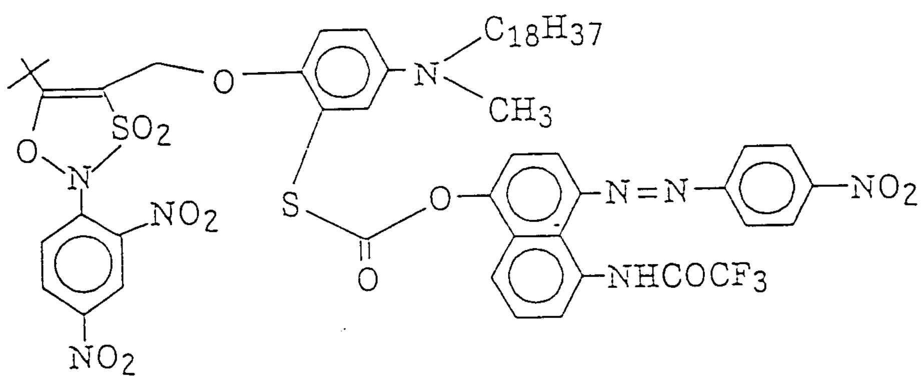

27

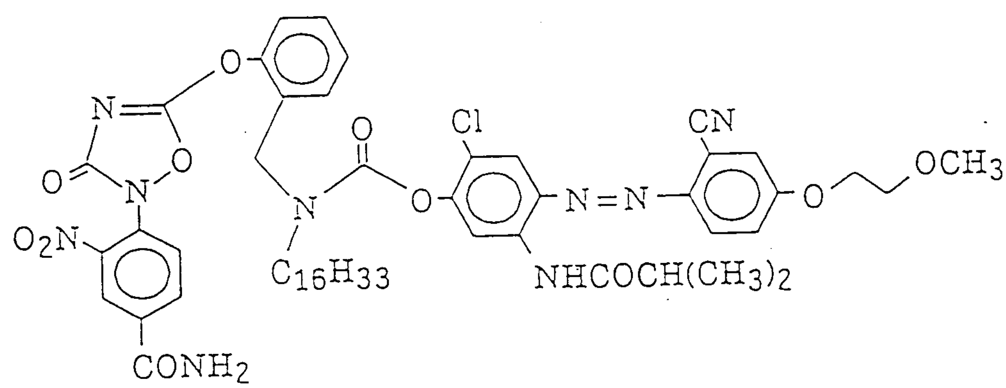

28

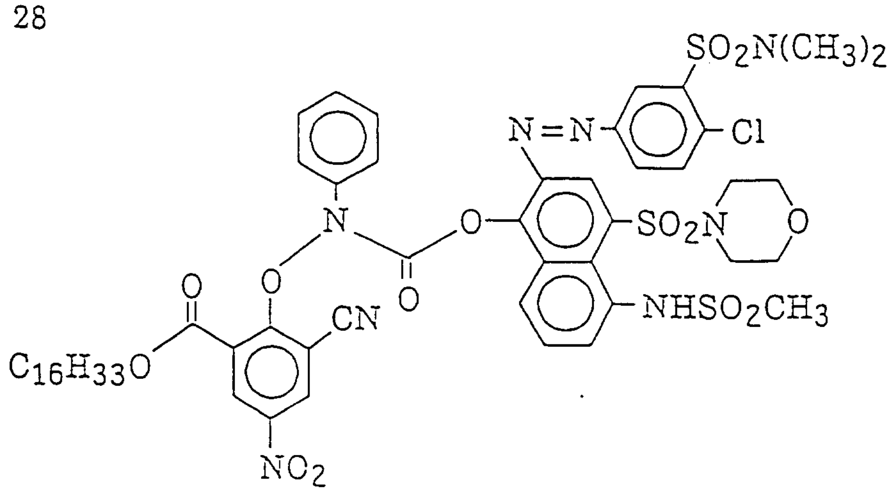

29

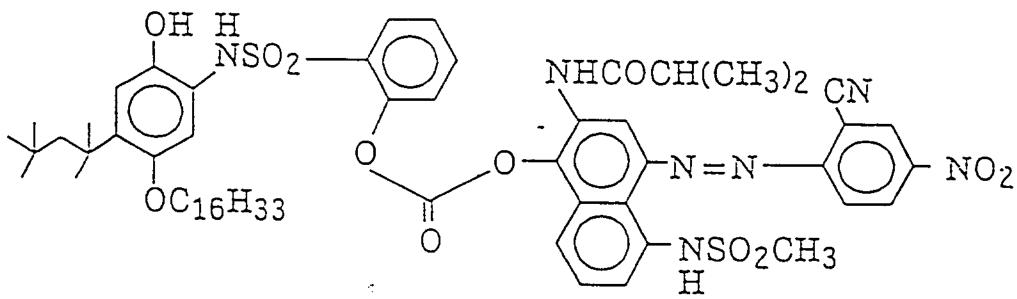

30

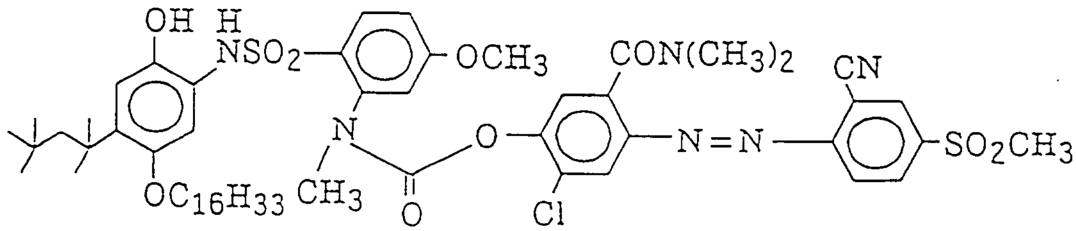

31

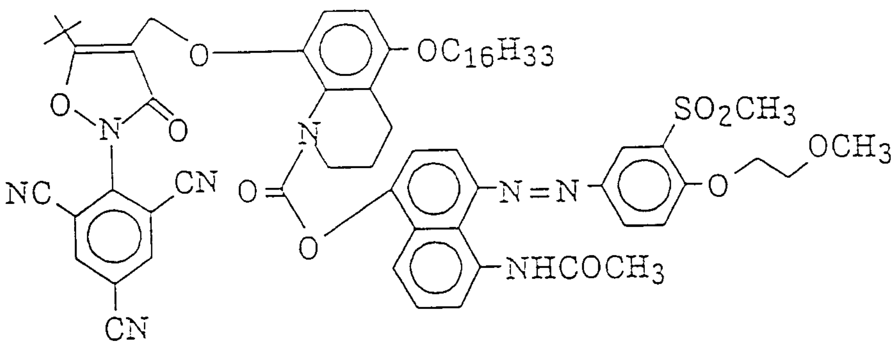

32

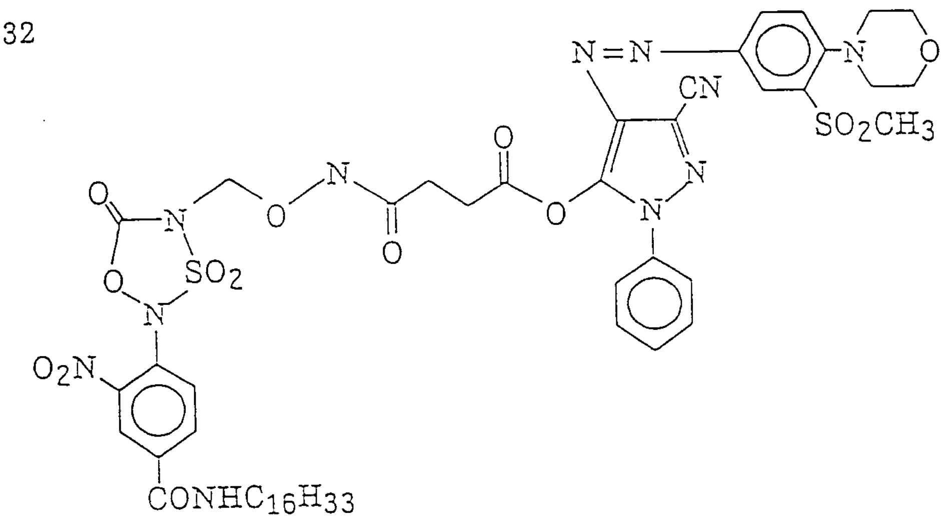

33

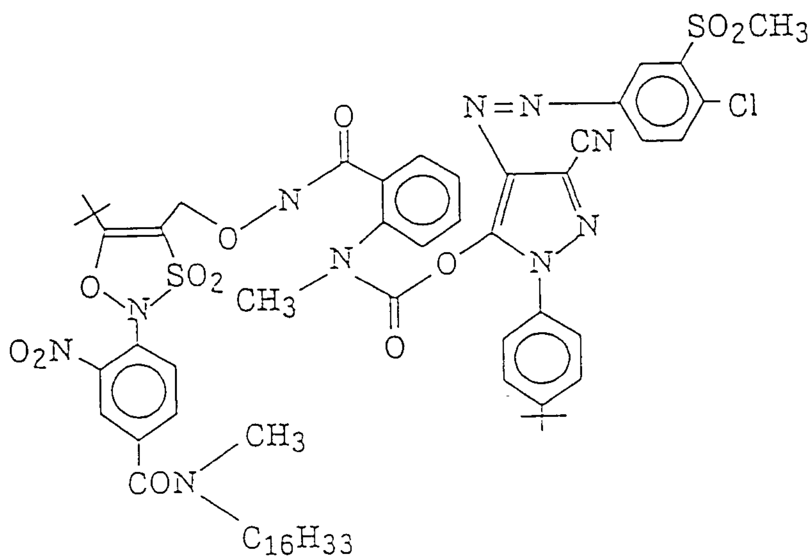

34

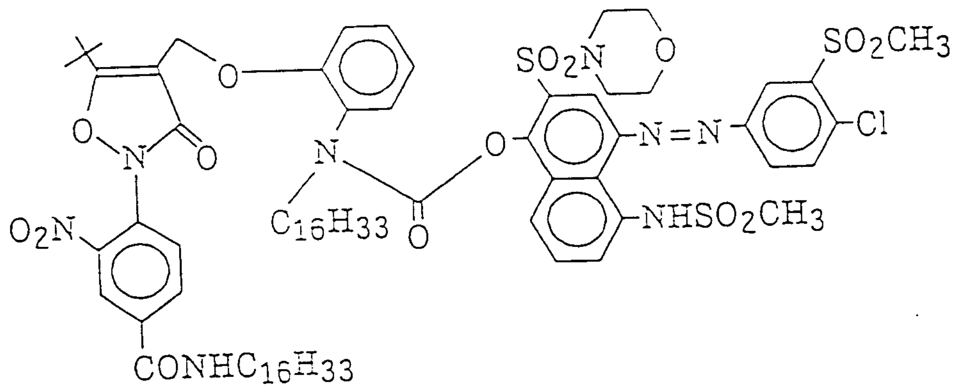

35

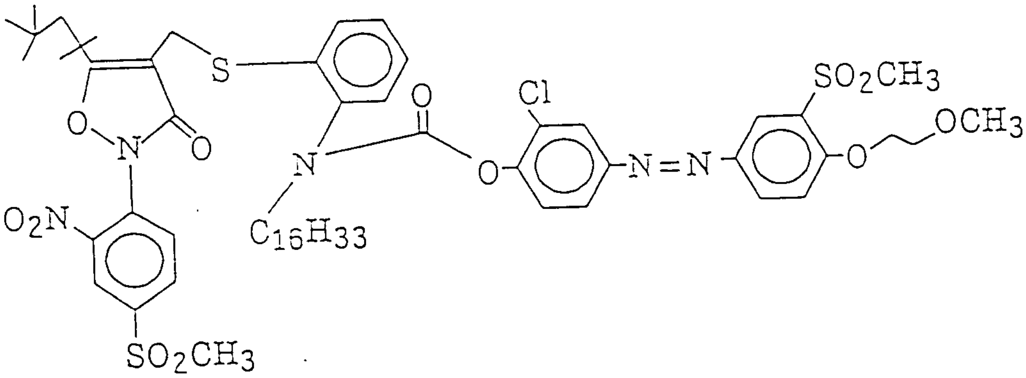

36

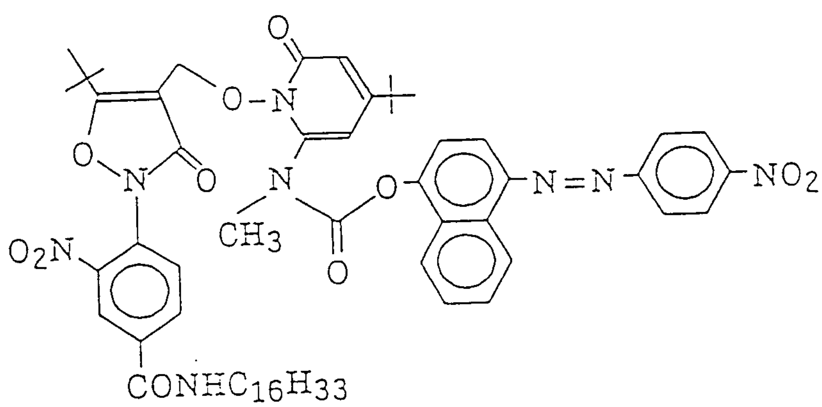

37

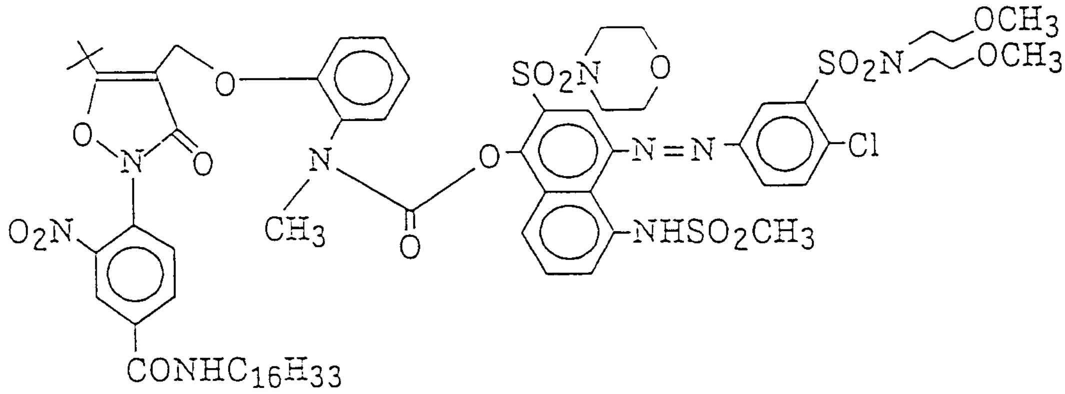

38

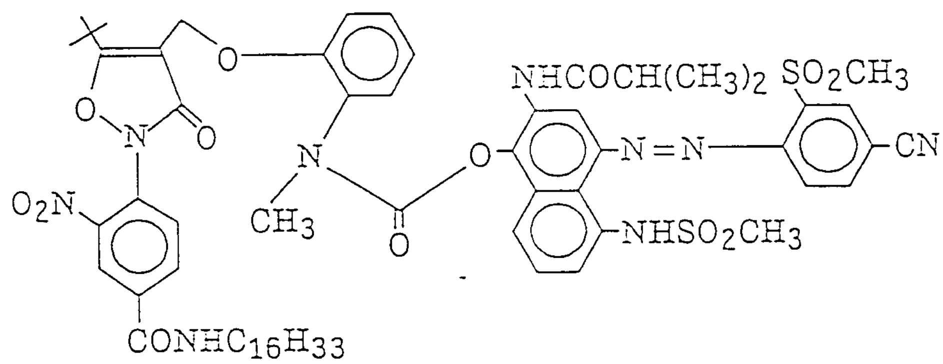

39

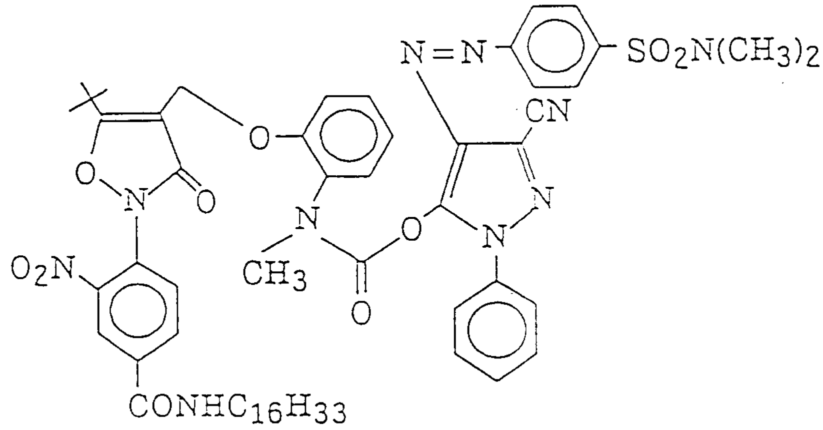

Synthesis examples for the compounds of the present invention are given below.

I. Synthesis of Exemplified Compound 37

I-1 Synthesis of the nucleophilic group precursor portion (introduction of a protective group on nitrogen)

Ortho (N-methylamino) phenol 24.6 g was dissolved in acetonitrile 250 ml and di-t-butyl carbonate 44 g was slowly added thereto at room temperature, followed by carrying out a reaction for 6 hours. After finishing the reaction, 1N-hydrochloric acid 500 ml was added and further ethyl acetate 500 ml was added for an extraction. After the extract was dried on anhydrous magnesium sulfate, it was concentrated in a rotary evaporator under reduced pressure, whereby the nucleophilic group precursor portion Compound (1) 38.4 g was obtained in the form of a pale yellow liquid. Yield: 92%.

I-2 Synthesis of Compound (2)

A Redox Principal Nucleus (A) 21 g, potassium carbonate 10.2 g, and sodium iodide 1.6 g were dissolved and suspended in acetone 100 ml and Compound (1) 7.6 g was added thereto, followed by heating at reflux for 5 hours. After finishing the reaction, water 200 ml and ethyl acetate 300 ml were added for an extraction. The organic phase so extracted was dried on anhydrous magnesium sulfate and was then concentrated in a rotary evaporator under reduced pressure, whereby Compound (2) 27 g was obtained in the form of a pale yellow oily material. Yield: 99%.

I-3 Synthesis of Compound (3) by deblocking of Compound 2

Compound (2) 7.5 g was dissolved in a 3 M-hydrochloric acid solution 100 ml of ethyl acetate and allowed to react over night. After finishing the reaction, water 100 ml was added to the reaction solution for an extraction. The organic phase so extracted was dried on anhydrous magnesium sulfate and was then concentrated in a rotary evaporator under reduced pressure, whereby Compound (3) 5.8 g was obtained in the form of a pale yellow oily material. Yield: 90%.

I-4 Synthesis of the temporarily shorter wavelength shifted dye-providing compound (Exemplified Compound No. 37)

Magenta Dye (B) 7.2 g and diisopropylethylamine 1.3 g were dissolved in methylene chloride 50 ml and a methylene chloride solution 10 ml of trichloromethyl chloroformate 1.2 ml was added dropwise thereto while cooling with ice. Further, the reaction temperature was raised to room temperature and stirring was applied for 2 hours to thereby prepare a formic chloride of the dye. Further, diisopropylethylamine 1.3 g and Compound (3) 6.45 g were added to react them at room temperature for one day and night. After finishing the reaction, water 100 ml and methylene chloride 50 ml were added to the reaction solution for an extraction. The organic phase so extracted was dried on anhydrous magnesium sulfate and was then concentrated in a rotary evaporator under reduced pressure. The concentrate was subjected to silica column chromatography, whereby the temporarily shorter wavelength-shifted dye-providing compound (exemplified Compound 37) 9.9 g was obtained from a methylene chloride - ethyl acetate 5 : 1 elution fraction in the form of an orange color crystal. Yield: 70%.

Compound (1)

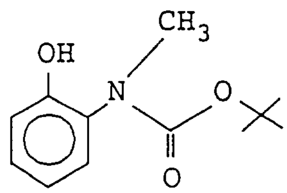

Redox Principal Nucleus (A)

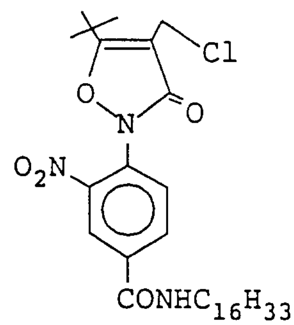

Compound (2)

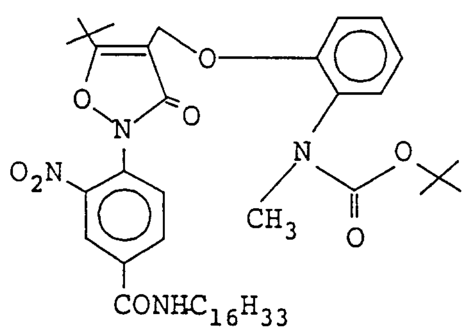

Magenta Dye (B)

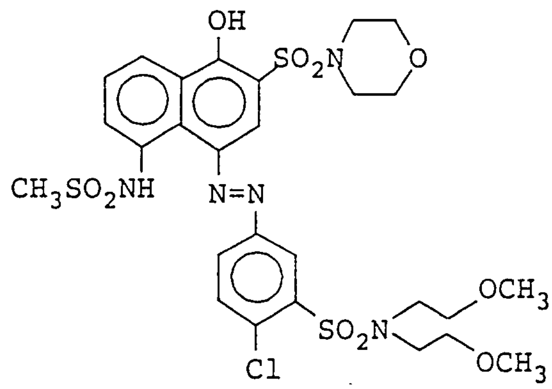

Compound (3)

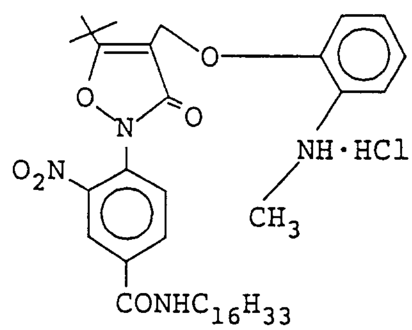

The compounds of the present invention can be used as dye-providing compounds for any of the three colors of yellow, magenta and cyan. Further, the compounds of the present invention may be used as dye-providing materials for all of the three colors in a light-sensitive material, or the temporarily shorter wavelength-shifted compounds of the present invention may be used only for any one color and non-temporarily shorter wavelength-shifted dye-providing compounds may be used for the other two colors, or the non-temporarily shorter wavelength-shifted dye-providing compounds may be used only for any one color and the dye-providing compounds of the present invention may be used for the other two colors.

A compound of the present invention may be incorporated in any layer of the light-sensitive material and is preferably incorporated in a light-sensitive silver halide emulsion layer.

An electron transfer agent (ETA) can be incorporated in a light-sensitive material according to the present invention in order to accelerate silver development.

Preferred ETA compounds are represented by the following Formulas (A) and (B):

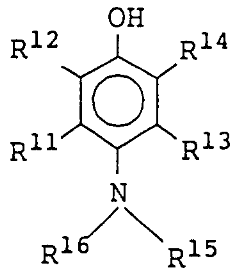

(A)

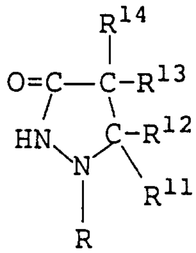

(B)

In Formulas (A) and (B), R represents an aryl group; and $R^{11}$, $R^{12}$, $R^{13}$, $R^{14}$, $R^{15}$ and $R^{16}$ each represents a hydrogen atom, a halogen atom, an acylamino group, an alkoxy group, an alkylthio group, an alkyl group, or an aryl group, and they may be the same as or different from each other.

Examples of the aryl group represented by R in Formulas (A) and (B) include, for example, phenyl, naphthyl, tolyl, and xylyl. These groups may be substituted. They may be an aryl group substituted with, for example, a halogen atom (e.g., a chlorine atom or a bromine atom), an amino group, an alkoxy group, an aryloxy group, a hydroxy group, an aryl group, a carbonamide group, a sulfonamide group, an alkanoyloxy group, a benzoyloxy group, a ureido group, a carbamate group, a carbamoyloxy group, a carbonate group, a carboxyl group, a sulfo group, and an alkyl group (e.g., methyl, ethyl, and propyl).

The alkyl group represented by $R^{11}$, $R^{12}$, $R^{13}$, $R^{14}$, $R^{15}$ and $R^{16}$ in Formulas (A) and (B) is an alkyl group having a carbon number of 1 to 10 (for example, methyl, ethyl, propyl, and butyl) and this alkyl group may be substituted with a hydroxyl group, an amino group, a sulfo group, or a carboxy group. There can be used as the aryl group represented by $R^{11}$ to $R^{16}$, phenyl, naphthyl, xylyl, and tolyl. These aryl groups may be substituted with a halogen atom (e.g., a chlorine atom or a bromine atom), an alkyl group (e.g., methyl, ethyl, and propyl), a hydroxy group, an alkoxy group (e.g., methoxy and ethoxy), a sulfo group, and a carboxy group.

In the present invention, the compounds represented by Formula (B) are particularly preferred. In Formula (B), $R^{11}$, $R^{12}$, $R^{13}$, and $R^{14}$ each are preferably a hydrogen atom, an alkyl group having a carbon number of 1 to 10, a substituted alkyl group having a carbon number of 1 to 10, or a substituted or unsubstituted aryl group. More preferably, $R^{11}$, $R^{12}$, $R^{13}$, and $R^{14}$ are each a hydrogen atom, a methyl group, a hydroxymethyl group, a phenyl group, or a phenyl group substituted with a hydrophilic group such as a hydroxy group, an alkoxy group, a sulfo group, and a carboxyl group.

Specific examples of ETA compounds represented by Formulas (A) and (B) are shown below.

(ETA-1)

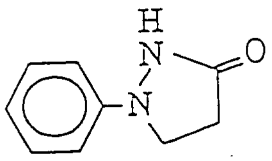

(ETA-2)

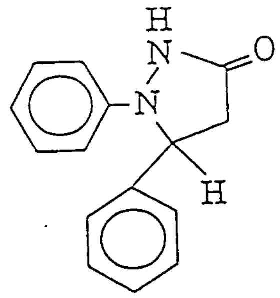

(ETA-3)

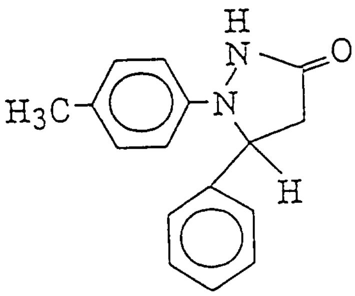

(ETA-4)

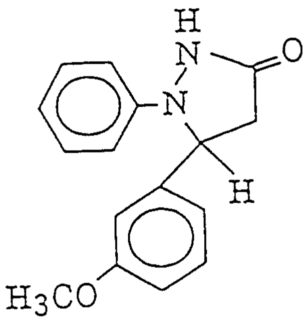

(ETA-5)

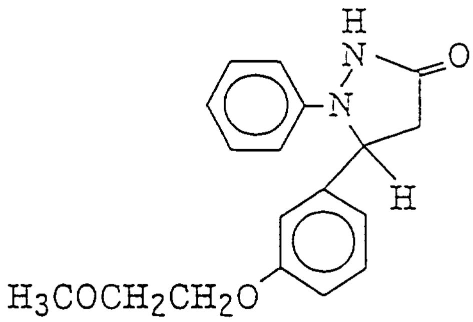

(ETA-6)

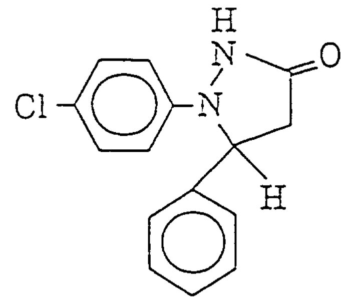

(ETA-7)

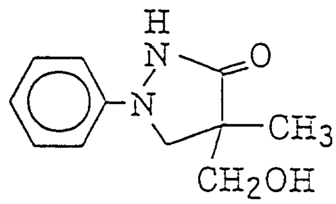

(ETA-8)

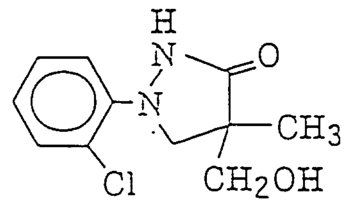

(ETA-9)

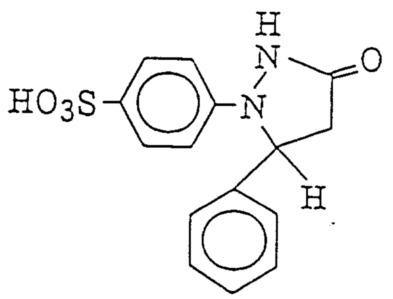

(ETA-10)

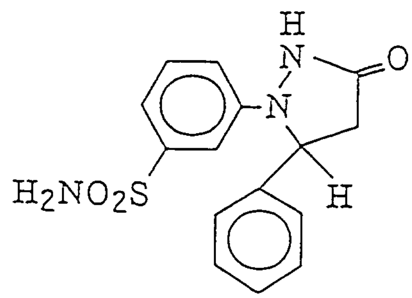

An ETA precursor may be incorporated in a photographic material of the present invention. The expression "ETA precursor" as used in the present specification means a compound which does not have a developing action during storage before using the light-sensitive material but which can release an ETA by the action of a suitable activating agent (for example, a base and a nucleophilic agent) or heating.

In particular, an ETA precursor for use in the present invention does not function as an ETA before development since a reactive functional group is blocked by a blocking group, but a cleavage of the blocking group under alkaline conditions or by heating can allow it to function as an ETA. Examples of ETA precursors suitable for use in the present invention include a 2-or 3-acyl derivative of 1-phenyl-3-pyrazolidinone, a 2-aminoalkyl or hydroxyalkyl derivative, a metal salt of hydroquinone and catechol (e.g., lead, cadmium, calcium, and barium), a halogenated acyl derivative of hydroquinone, an oxazine or bisoxazine derivative of hydroquinone, a lactone type ETA precursor, a hydroquinone precursor having a quaternary ammonium group, and a cyclohexa-2-ene-1,4-dione type compound as well as a compound which releases an ETA by an electron transfer reaction, a compound which releases an ETA by an intermolecular nucleophilic displacement reaction, an ETA precursor blocked by a phthalide group, and an ETA precursor blocked by an indomethyl group.

ETA precursors suitable for use in the present invention are known compounds and there can be used the developing agent precursors described in, for example, U.S. Patents 767,704, 3,241,967, 3,246,988, 3,295,978, 3,462,266, 3,586,506, 3,615,439, 3,650,749, 4,209,580, 4,330,617, and 4,310,612, British Patents 1,032,701, 1,231,830, 1,258,924, and 1,346,920, and JP-A-57-40245, JP-A-58-1139, JP-A-58-1140, JP-A-59-178458, JP-A-59-182449, and JP-A-59-182450.

The ETA precursors of 1-phenyl-3-pyrazolidinones described in JP-A-59-178458, JP-A-59-182449, and JP-A-59-182450 are particularly preferred.

The electron transfer agent or precursor thereof is incorporated into a light-sensitive element or a processing composition.

When the shifted dye-providing compound of the present invention is a reducible compound, a reducing agent is used in or with the photographic material of the invention. The reducing agent may be incorporated into any layer in a light-sensitive material or it may be supplied from an external source. Preferably the reducing agent is incorporated into the same layer as that containing the shifted dye-provding compound.

Examples of reducing agents suitable for use in the present invention include the reducing agents and reducing agent precursors described in columns 49 to 50 of U.S. Patent 4,500,626, columns 30 to 31 of U.S. Patent 4,483,914, U.S. Patents 4,330,617 and 4,590,152, JP-A-60-140335, pp. 17 to 18, JP-A-57-40245, JP-A-56-138736, JP-A-59-178458, JP-A-59-53831, JP-A-59-182449, JP-A-59-182450, JP-A-60-119555, JP-A-60-128436 to JP-A-60-128439, JP-A-60-198540, JP-A-60-181742, JP-A-61-259253, JP-A-62-244044, and JP-A-62-131253 to JP-A-62-131256, and European Patent 220,746A2, pp. 78 to 96.

Suitable reducing agents include compounds represented by the following Formulas (C) and (D):

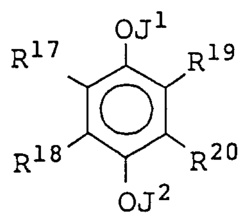

(C)

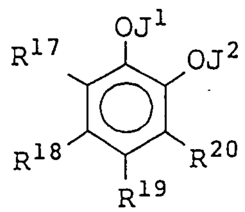

(D)

In Formulas (C) and (D), $J^1$ and $J^2$ each represents a hydrogen atom or a protective group for a phenolic hydroxy group capable of deblocking with a nucleophilic reagent.

Examples of nucleophilic reagents include an anionic reagent such as $OH^-$, $RO^-$ (R: an alkyl group, an aryl group, and others), hydroxamic acid anions, $SO_3^{2-}$, and compounds having an unshared electron pair, such as primary or secondary amines, hydrazines, hydroxylamines, alcohols, and thiols. Preferred examples of $J^1$ and $J^2$ include a hydrogen atom, an acyl group, an alkylsulfonyl group, an arylsulfonyl group, an alkoxycarbonyl group, an aryloxycarbonyl group, a dialkylphosphoryl group, a diarylphosphoryl group, or the protective groups disclosed in JP-A-59-197037 and JP-A-59-20105. If possible, $J^1$ and $J^2$ may be combined with $R^{17}$, $R^{18}$, $R^{19}$ and $R^{20}$ to form a ring. $J^1$ and $J^2$ may be the same as or different from each other.

$R^{17}$, $R^{18}$, $R^{19}$ and $R^{20}$ each represents a hydrogen atom, an alkyl group (an alkyl group which may be substituted, for example, methyl, ethyl, n-butyl, cyclohexyl, n-octyl, allyl, sec-octyl, tert-octyl, n-dodecyl, n-pentadecyl, n-hexadecyl, tert-octadecyl, 3-hexadecanoylaminophenylmethyl, 4-hexadecylsulfonylamino-phenylmethyl, 2-ethoxycarbonylethyl, 3-carboxypropyl, N-ethylhexadecylsulfonylaminomethyl, and N-methyldodecylsulfonylaminoethyl); an aryl group (an aryl group which may be substituted, for example, phenyl, 3-hexadecyloxyphenyl, 3-methoxyphenyl, 3-sulfonphenyl, 3-chlorophenyl, 2-carboxyphenyl, and 3-decanoylaminophenyl); an alkylthio group (an alkylthio group which may be substituted, for example, n-butylthio, methylthio, tert-octylthio, n-dodecylthio, 2-hydroxyethylthio, n-hexadecylthio, and 3-ethoxycarbonylpropylthio); an arylthio group (an arylthio group which may be substituted, for example, phenylthio, 4-chlorophenylthio, 2-n-octyloxy-5-t-butylphenylthio, 4-dodecyloxyphenylthio, and 4-hexadecanoylaminophenylthio); a sulfonyl group (an aryl- or alkylsulfonyl group which may be substituted, for example, methanesulfonyl, butanesulfonyl, p-toluyenesulfonyl, 4-dodecyloxyphenylsulfonyl, and 4-acetylaminophenylsulfonyl); a sulfo group; a halogen atom (for example, a fluorine atom, a chlorine atom, a bromine atom, an iodine atom); a cyano group; a carbamoyl group (a carbamoyl group which may be

substituted, for example, methylcarbamoyl, diethylcarbamoyl, 3-(2,4-di-t-pentylphenyloxy) propylcarbamoyl, cyclohexylcarbamoyl, and di-n-octylcarbamoyl); a sulfamoyl group (a sulfamoyl group which may be substituted, for example, diethylsulfamoyl, di-n-octylsulfamoyl, n-hexadecylsulfamoyl, and 3-isohexadecanoylaminophenylsulfamoyl); an amide group (an amide group which may be substituted, for example, acetamide, iso-butyloylamino, 4-tetra-decyloxyphenylbenzamide, and 3-hexadecanoylaminobenzamide); an imide group (an imide group which may be substituted, for example, succinimide, 3-lauryl succinimide, and phthalimide); a carboxyl group; and a sulfonamide group (a sulfonamide group which may be substituted, for example, methanesulfonamide, octanesulfonamide, hexadecanesulfonamide, benzenesulfonamide, toluenesulfonamide, and 4-lauryloxybenzenesulfonamide), provided that the total carbon number of $R^{17}$ to $R^{20}$ is 8 or more. Further, $R^{17}$ and $R^{18}$, and/or $R^{19}$ and $R^{20}$ in Formula (C), and $R^{17}$ and $R^{18}$, $R^{18}$ and $R^{19}$, and/or $R^{19}$ and $R^{20}$ in Formula (D) may be combined with each other to form a saturated or unsaturated ring.

Of the reducing agents represented by the above Formulas (C) and (D), those reducing agents are preferred in which at least two of $R^{17}$ to $R^{20}$ are substituents other than a hydrogen atom. Particularly preferred are compounds in which at least one of $R^{17}$ and $R^{18}$ and at least one of $R^{19}$ and $R^{20}$ are substituents other than a hydrogen atom.

A plurality of reducing agents may be used and a reducing agent and a precursor thereof may be used in combination.

Specific examples of reducing agents are given below but the reducing agents are not limited to these compounds.

(ED-1)

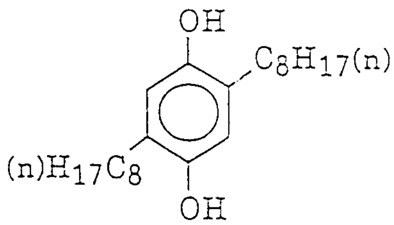

(ED-2)

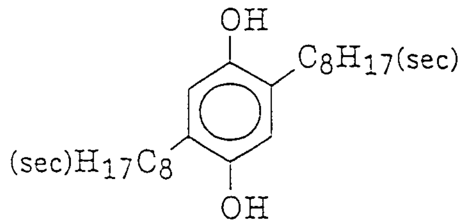

(ED-3)

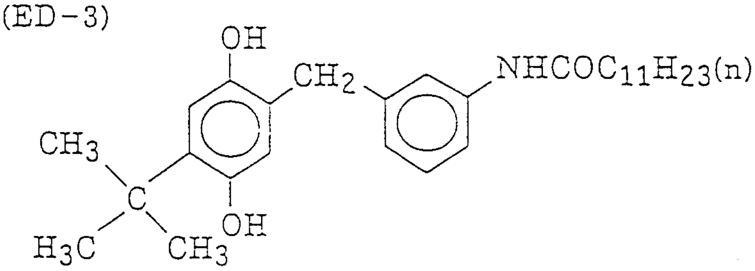

(ED-4)

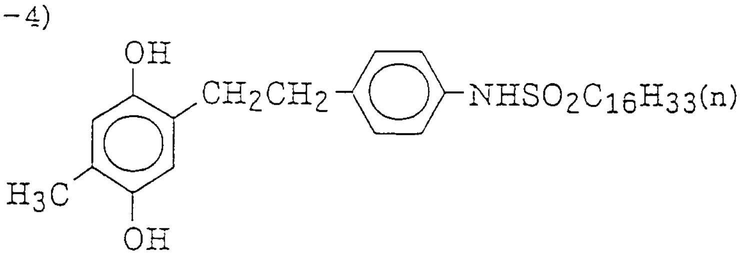

(ED-5)

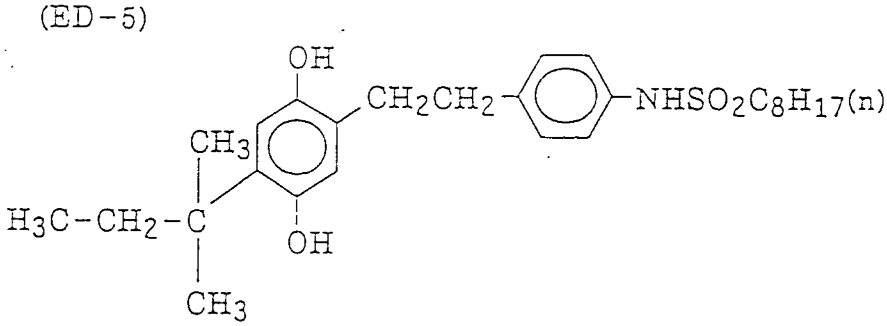

(ED-6)

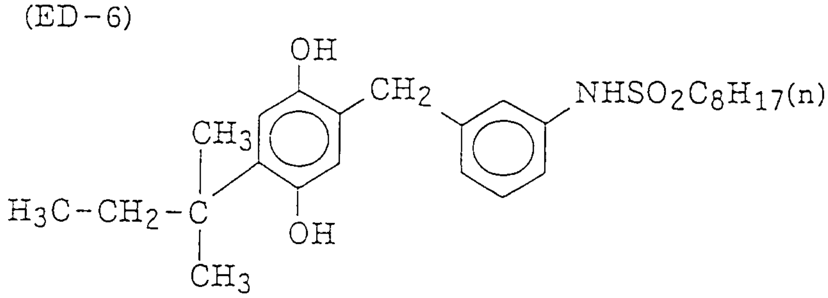

(ED-7)

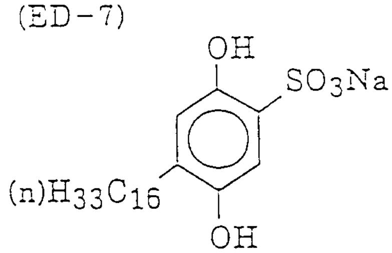

(ED-8)

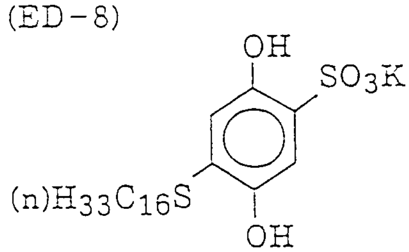

(ED-9)

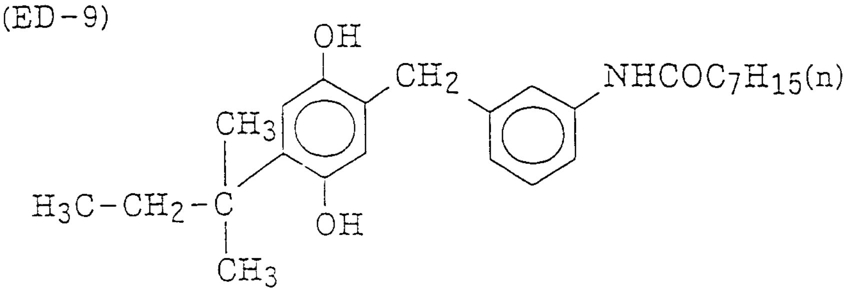

(ED-10)

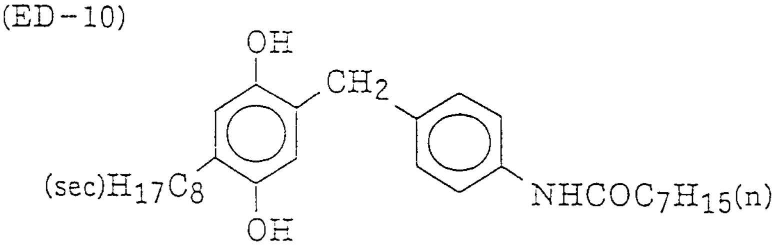

(ED-11)

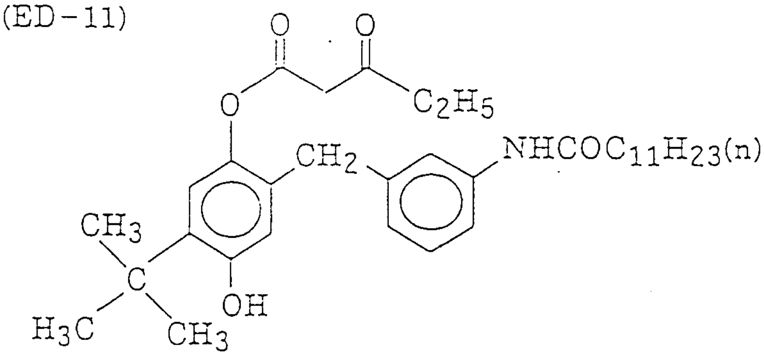

(ED-12)

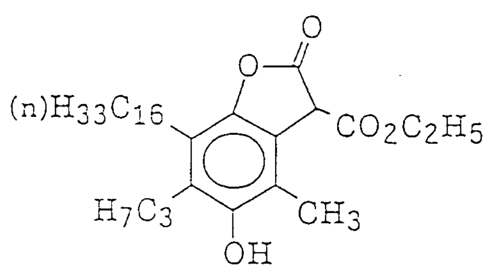

Silver halide compositions suitable for use in the present invention are described at p. 48, line 8 to p. 58, line 15 of JP-A-1-166038, which description is incorporated herein by reference. The cited portion of JP-A-1-166038 also describes the following emulsions, grains, sensitization techniques, and so on, which are suitable for use in the context of the present invention, monodispersed emulsions, sizes and formations of silver halide grains, latent image type silver halide emulsions, spectral sensitization of silver halides,

protective colloids, surface active agents, anti-fogging agents, stabilizers, compounds added to increase sensitivity and/or contrast, development accelerators, polymer dispersions to improve dimensional stability, and various other additives.

Compounds of Formula (I) of the present invention which can release a diffusible dye are preferably used for heat developable silver halide light-sensitive materials in which a mobile dye is formed by heat development and transferred to a dye fixing layer, as described in, for example, JP-A-58-149046, JP-A-59-154445, JP-A-59-165054, JP-A-59-180548, JP-A-59-218443, and JP-A-60-133449, U.S. Patents 4,503,137, 4,474,867, 4,483,914, 4,455,363, and 4,500,626, and JP-A-60-79709.

Where a compound capable of releasing a diffusible dye is used in a heat developable light-sensitive element, an organic metal salt can also be used as an oxidizing agent in combination with the light-sensitive silver halide. Of such organic metal salts, an organosilver salt is particularly preferably used.

Organic compounds which can be used for forming the above organosilver salt oxidizing agent include benzotriazoles, aliphatic acids and other compounds as described in U.S. Patent 4,500,626, columns 52 to 53. Further, also useful are silver salts of carboxylic acids having an alkynyl group, such as silver phenylpropionic acid, as described in JP-A-60-113235, and acetylene silver as described in JP-A-61-249044. The silver salts may be used in combinations of two or more kinds.

The above organosilver salts can be used either alone or in combinations in the amount of 0.01 to 10 mole, preferably 0.01 to 1 mole per mole of light-sensitive silver halide. The total coated amount of light-sensitive silver halide and organosilver salt is suitably 50 mg to 10 $g/m^2$ in terms of converted silver.

In the present invention, various anti-fogging agents and photographic stabilizers can be used. Examples thereof include azoles and azaindenes as described in RD 17643 (1978), pp. 24 to 25, carboxylic acids containing nitrogen and phosphoric acids as described in JP-A-59-168442, the mercapto compounds and metal salts thereof described in JP-A-59-111636, and the acetylene compounds described in JP-A-62-87957.

A hydrophilic compound is preferably used as a binder for the layers of the light-sensitive element and the dye fixing element. Example thereof include the compounds described at pages 26 to 28 of JP-A-62-253159. To be specific, a transparent or translucent hydrophilic binder is preferred. Examples include gelatin and gelatin derivatives, polysaccharides (including cellulose derivatives, starches, gum arabic, dextran, and pluran) and synthetic high molecular weight compounds such as polyvinyl alcohols, polyvinyl pyrrolidones, acrylamide polymers, and others. Further, there can be used a highly water absorptive polymer as described in JP-A-62-245260, that is, a homopolymer of a vinyl monomer having a -COOM or $-SO_3M$ group (where M represents a hydrogen atom or an alkali metal), or a copolymer of two such vinyl monomers or a copolymer of such a vinyl monomer with another vinyl monomer (for example, sodium methacrylate, ammonium methacrylate and Sumika Gel L-5H manufactured by Sumitomo Chemical Co., Ltd.). These binders can be used in combinations of two or more kinds.

Where a system in which a trace amount of water is supplied to carry out a heat development is used, the use of the above highly water absorptive polymer makes it possible to absorb water rapidly. Further, the use of a highly water absorptive polymer can prevent a dye from retransferring from a dye fixing element to other layers after transfer.

In the present invention, the coated amount of binder is preferably 20 $g/m^2$ or less, particularly 10 $g/m^2$ or less, and more preferably 7 $g/m^2$ or less.

Various polymer latexes can be incorporated into the constituting layers (including a back layer) of the light-sensitive element or of the dye fixing element to improve the film's physical properties such as dimensional stability, curling prevention, sticking prevention, cracking prevention, and pressure sensitization or desensitization prevention. To be specific, any of the polymer latexes described in JP-A-62-245258, JP-A-62-136648 and JP-A-62-110066 can be used. The use of a polymer latex having a low glass transition point (40 °C or lower) as a mordant layer can prevent cracking of the mordant layer and the use of a polymer latex having a high glass transition point can provide a curling prevention effect.

Compounds known in the art of heat developable light-sensitive materials can be used as reducing agents for use in the present invention. Further, the dye-providing compound having a reducing function, which will be described later, are included therein (in this case, the other reducing agents can also be used in combination). Reducing agents precursor which have no reducing function by themselves but which reveals their reducing function by the action of a nucleophilic agent or heat in the course of development can also be used.

The present invention can be applied to diffusion transfer type color light-sensitive materials which are subjected to development processing in an alkali processing composition.

The processing composition used in the present invention is evenly spread on a light-sensitive element after the light-sensitive element is exposed and a light-sensitive layer is developed with the components

contained in the processing composition. For this purpose, the composition should contain alkali, a thickener, a light shielding agent, and an electron transfer agent (a developing agent), and should further contain a development accelerator and a development inhibitor for controlling development, and an anti-oxidation agent for preventing deterioration of the developing agent. The composition can contain a light shielding agent, if necessary.

The alkali should provide a pH of 12 to 14. Examples of suitable alkalis include hydroxides of alkali metals (for example, sodium hydroxide, potassium hydroxide and lithium hydroxide), phosphates of alkali metals (for example, potassium phosphate), guanidines, and hydroxides of quaternary amines (for example, tetramethylammonium hydroxide). Of these, potassium hydroxide and sodium hydroxide are preferred.

A thickener is necessary for evenly spreading the processing solution, maintaining adhesion between the light-sensitive element and the image-receiving element, and preventing processing solution components from remaining on the surface of the image-receiving element after peeling.

Examples of suitable thickeners include polyvinyl alcohols, hydroxyethyl cellulose, and alkali metal salts of carboxymethyl cellulose. Hydroxyethyl cellulose and sodium carboxymethyl cellulose are preferred.

Where the image-receiving element is provided on a transparent support having no light-shielding function, it can contain a light shielding agent.

Either a dye or a pigment or a combination thereof can be used as a light shielding agent provided that diffusion of the light shielding agent to the image-receiving element does not generate stain. Carbon black is a typical example of a light shielding agent and in addition thereto, the combination of titanium white and a dye can be used as well. This dye may be a temporary light shielding dye which becomes colorless after a prescribed time.

When REDOX is a reducible group, the combination of an electron transporting agent and an electron donating compound as known in the art, can be used. The electron donating agent reduces the reducible REDOX to release a diffusible dye. The electron transporting agent reduces an exposed silver halide and, at the same time, the resultant oxidation product oxidizes the electron donating compound. Therefore, a diffusible dye is not released in an exposed region and a diffusible dye is released in an unexposed region. The image formation reaction, electron trnasporting agents and electron donating compounds are described in detail in U.S. Patent 4,783,396.

In the present invention, the addition amount of the non-diffusible reducing agent is about 0.01 to about 20 mole, particularly preferably about 0.1 to about 10 mole per mole of silver.

Image stabilizing compounds and development accelerating compounds can be used in the present invention. Preferred examples of such compounds are described at columns 51 to 52 of U.S Patent 4,500,626.

In a system in which an image is formed by diffusion transfer of a dye, a dye fixing element is used together with a light-sensitive element. The dye fixing element may be of the form in which the dye fixing element is independently coated on a support different from that for the light-sensitive element, or it may be of the form in which it is coated on the same support as that for the light-sensitive element. With respect to the relationships of the light-sensitive element to the dye fixing element, support and white color reflection layer, the relationships described at the 57th column of U.S. Patent 4,500,626 can be applied as well to the present invention.

The dye fixing element preferably used in the present invention has at least one layer containing a mordant and a binder. The compounds known in the photographic field can be used as the mordant and specific examples thereof include the mordants described at columns 58 to 59 of U.S. Patent 4,500,626 and pages 32 to 34 of JP-A-61-88256, and the compounds described in JP-A-62-244043 and JP-A-62-244036. Further, the dye-receivable high molecular weight compounds described in U.S. Patent 4,463,079 may be used.

The dye fixing element can be provided with one or more auxiliary layers such as a protective layer, a peeling layer, and an anti-curling layer as desired. In particular, the provision of a protective layer is useful.

The layers of the light-sensitive element and the dye fixing element may include a plasticizer, a sliding agent, or a high boiling solvent as a peeling improver for the light-sensitive element and dye fixing element. To be specific, the compounds described in JP-A-62-253159, p. 25 and JP-A-62-245253 may be used for these purposes.

Further, various silicon oils (such as silicon oils obtained from a dimethyl silicon oil, and modified silicon oils obtained by introducing various organic groups into dimethyl siloxane) can be used for the above purposes. Example of suitable silicon oils include various modified silicon oils as described in "Modified Silicon Oil" technical literature P6-18B, particularly carboxy-modified silicon oils (trade name: X-22-3710).

The silicon oils described in JP-A-62-215953 and JP-A-62-23687 are effective as well.

An anti-fading agent may be used for the light-sensitive element and dye fixing element. Examples of anti-fading agents include anti-oxidation agents, UV absorbers and certain types of metal complexes.

More specific examples of anti-oxidation agents include chroman compounds, coumarane compounds, phenol compounds (for example, hindered phenols), hydroquinone derivatives, hindered amine derivatives, and spiroindane compounds. Further, the compounds described in JP-A-61-159644 are effective as well.

Examples of UV absorbers include benzotriazole compounds (U.S. Patent 3,533,794), 4-thiazolidone compounds (U.S. Patent 3,352,681), benzophenone compounds (JP-A-56-2784), and the compounds described in JP-A-54-48535, JP-A-62-136641 and JP-A-61-88256. Further, the UV absorptive polymers described in JP-A-62-260152 are effective as well.

Examples of suitable metal complexes include the compounds described in U.S. Patent 4,241,155, in columns 3 to 36 of U.S. Patent 4,245,018, and in columns 3 to 8 of U.S. Patent 4,254,195, and in JP-A-62-174741, JP-A-61-88256 (pages 27 to 29), JP-A-62-234103, JP-A-62-31096 and JP-A-62-230596.

Examples of useful anti-fading agents are described in JP-A-62-215272 (pages 125 to 137).

The anti-fading agent used for preventing fading of the dye transferred to the dye fixing element may be incorporated in advance into the dye fixing element or it may be supplied to the dye fixing element from outside the light-sensitive element.

The above anti-oxidation agents, UV absorbers and metal complexes may be used in combinations.

Additional additives and the like suitable for use in the present invention are described at page 69, line 13 to page 73, line 9 of JP-A-1-166038, including image formation accelerators, fluorescent whitening agents capable of being used for a light-sensitive element and a dye fixing element, hardeners capable of being used for the layers of the light-sensitive element and dye fixing element, coating aids, organic fluorinated compounds, matting agents, heat solvents, defoamers, anti-microbial agents, and colloidal silicas.

In a system in which a heat development and a transfer of a dye are carried out simultaneously in the presence of a small amount of water, a base and/or a base precursor are preferably incorporated into a dye fixing element to increase the preservability of the light-sensitive element.

There can also be used as a base precursor, the combination of a scarcely soluble metal compound and a compound (called as a complex forming compound) capable of forming a complex with a metal ion in the scarcely soluble metal compound, as described in European Patent Publication 210,660, and compounds generating a base by electrolysis, as described in JP-A-61-232451. The former method is particularly effective. These scarcely soluble compounds and complex forming compounds are advantageously added separately to the light-sensitive element and dye fixing element.

Various development stoppers can be used for the light-sensitive element and/or dye fixing element of the present invention for the purpose of obtaining a constant image not subject to variations of processing temperature and processing time in the development.

The development stopper is an optimum developing solution which contains a compound which neutralizes or reacts with a base to lower the base concentration in a layer to stop the development, or a compound which controls development by its interaction with silver or a silver salt. Specific examples of compounds suitable for use in development stoppers include acid precursors which release an acid upon heating, electrophilic compounds which cause a displacement reaction with a coexisting base upon heating, nitrogen-containing heterocyclic compounds, and mercapto compounds or precursors thereof. More details are described at pages 31 to 32 of JP-A-62-253159.

Further, a layer having a neutralization function can be used for the above purpose of development stopping. A neutralization timing layer can also be used to allow the neutralization function to occur optimally.

A layer having a neutralization function is a layer containing an acidic material in an amount sufficient to neutralize the alkali carried over from the processing composition. According to necessity, it may be of a multi-layer construction consisting of layers such as a neutralization speed controlling layer (a timing layer) and an adhesion strengthening layer. Preferred acidic materials include materials containing an acidic group having a pKa of about 9 or less (or a precursor group giving such an acidic group by a hydrolysis), and more. Preferred acidic material include higher fatty acids such as oleic acid, as described in U.S. Patent 2,983,606, polymers of acrylic acids, methacrylic acids or maleic acids and partial esters or acid anhydrides thereof, as disclosed in U.S. Patent 3,362,819; copolymers of acrylic acids and acrylic esters, as disclosed in French Patent 2,290,699; and latex type acidic polymers as disclosed in U.S. Patent 4,139,388 and Research Disclosure No. 16102 (1977).

Additional examples of preferred acidic materials include the acidic materials disclosed in U.S. Patent 4,088,493, and JP-A-52-153739, JP-A-53-1023, JP-A-53-4540, JP-A-53-4541, and JP-A-53-4542.

Specific examples of acidic polymers include copolymers of vinyl monomers such as ethylene, vinyl acetate and vinyl methyl ether and maleic anhydride and n-butyl esters thereof, copolymers of butyl acrylate and acrylic acid; cellulose acetate; and hydrogen phthalate.

The above acidic polymers can be used either singly or in combination with a hydrophilic polymer. Preferred hydrophilic polymers include polyacrylamides, polyvinylpyrrolidones, polyvinyl alcohols (including partially saponified polymers), carboxymethyl celluloses, hydroxymethyl celluloses, hydroxyethyl celluloses, and polymethyl vinyl ethers. Among them, polyvinyl alcohols are preferred.

Further, polymers other than the hydrophilic polymers, for example, cellulose acetate, may be mixed with the above acidic polymers.

The coated amount of the acidic polymer is adjusted according to the amount of alkali contained in the light-sensitive element. The equivalence ratio of the acidic polymer and alkali per unit area is preferably about 0.9 to about 2.0. Too small an amount of the acidic polymer changes the hue of the transferred dye or generates a stain on the background. Meanwhile, too much of the acidic polymer causes problems such as changes of the hue or deterioration of light resistance. The equivalence ratio is more preferably about 1.0 to about 1.3. In cases where an acidic polymer is mixed with a hydrophilic polymer, a too large or small amount of acidic polymer will deteriorate the quality of the resulting image. The weight ratio of the hydrophilic polymer to the acidic polymer is about 0.1 to about 10, preferably about 0.3 to about 3.0.

Additives can be incorporated in the layer having a neutralization function for various purposes. There can be added, for example, a hardener known well in the art for hardening this layer, and a polyhydric hydroxyl compound such as polyethylene glycol, polypropylene glycol and glycerine. In addition thereto, an oxidizing agent, a development inhibitor and a precursor thereof can be added according to necessity.

Useful additives for the timing layer used in combination with the neutralizing layer are, for example, a polymer for lowering the alkali permeability, such as polyvinyl alcohol, a partially acetalized compound of polyvinyl alcohol, and partially hydrolyzed polyvinyl acetate; a latex polymer for increasing the activation energy for alkali permeation, which is prepared by copolymerizing a small amount of a hydrophilic comonomer such as an acrylic acid monomer; and a polymer having a lactone ring.

The neutralization timing layer may have a single layer structure or a multi-layer structure.

It is possible as well to incorporate into the timing layer composed of these materials, for example, the development inhibitors and/or precursors thereof disclosed in U.S. Patent 4,009,029, German Patent Applications (OLS) 2,013,164 and 3,014,672, and JP-A-54-155837, the hydroquinone precursors disclosed in U.S. Patent 4,201,578, and other photographically useful additives or precursors thereof.

In a photographic material of the present invention, a peeling layer may be provided so that the light-sensitive element and the image-receiving element may be peeled apart after processing. Such a peeling layer has to be a layer which can be readily peeled after processing.

The contents of the descriptions at page 74, line 18 to page 77, line 7 of JP-A-1-166038 can be applied to the supports used for the light-sensitive element and image-receiving element used in the present invention, and to methods for exposing the light-sensitive element.

## EXAMPLES

Working examples of the invention are given below to illustrate the effects of the present invention in more detail.

### EXAMPLE 1

(1) Preparation of a silver halide emulsion
Preparation of a tabular silver bromide emulsion:

30 ml of a 0.40M silver nitrate aqueous solution and 30 ml of a 0.15M potassium bromide aqueous solution were added to a 0.8 weight% aqueous gelatin solution 1 liter containing 0.05M potassium bromide by a double jet method while stirring. During this time, the aqueous gelatin solution was maintained at 30°C. After finishing the addition, the temperature was raised to 75°C. Further, gelatin 30 g was added after the addition.

After finishing the above addition as the first step, a 0.4M aqueous silver nitrate solution 90 ml was added. Further, thereafter, silver nitrate 176 g was added at an accelerating flow rate (the final flow rate was twenty one times as much as the initial flow rate) over a period of 65 minutes. During this time, the pBr was maintained at 2.40.

After the grains thus formed (hereinafter called seed crystals) were washed by a conventional flocculation method and gelatin was added, the pH and pAg were adjusted to 6.5 and 8.0, respectively, at 40 ° C.

The seed crystal emulsion thus prepared contained silver halide 203 g per kg of the emulsion.

The above seed crystals were dispersed in an aqueous solution 3.7 liter containing 0.8 weight% gelatin and maintained at the temperature of 75 ° C and a pBr of 1.60. Then, silver nitrate 107 g was added while maintaining the pBr at 1.60.

Further, an ammonium thiocyanate salt was added and then silver nitrate was added so that the pBr became 2.70.

Then, the emulsion was cooled down to 35 ° C to wash it by a conventional flocculation method and after gelatin was added and the pH and pAg were adjusted to 6.5 and 8.5, respectively, at 40 ° C, the emulsion was stored in a cool and dark room. This tabular emulsion had an average projected area-corresponding circle diameter of 1.3 µm and an average thickness of 0.25 µm. This emulsion contained silver halide 213 g per kg of the emulsion.

Preparation of the emulsion for a red-sensitive layer:

The above tabular silver bromide emulsion 1 kg was heated to the temperature of 55 ° C and sodium benzenesulfonate was added, followed by adding the following Sensitizing Dyes S-1 and S-2. Further, 4-hydroxy-6-methyl-1,3,3a,7-tetrazaindene was added and then $Na_2SO_3$, $KAuCl_4$ and potassium thiocyanate were added to thereby carry out a chemical sensitization. When the maximum temperature was reached, the temperature was lowered to finish the chemical sensitization, whereby the emulsion for the red-sensitive layer was prepared.

Preparation of the emulsion for a green-sensitive layer:

The emulsion for the green-sensitive layer was prepared in the same manner as the emulsion for the red-sensitive layer except that the Sensitizing Dyes S-1 and S-2 were replaced with Sensitizing Dye S-3.

Preparation of the emulsion for a blue-sensitive layer:

The emulsion for the blue-sensitive layer was prepared in the same manner as the emulsion for the red-sensitive layer except that the Sensitizing Dyes S-1 and S-2 were replaced with Sensitizing Dye S-4.

Sensitizing Dye S-1

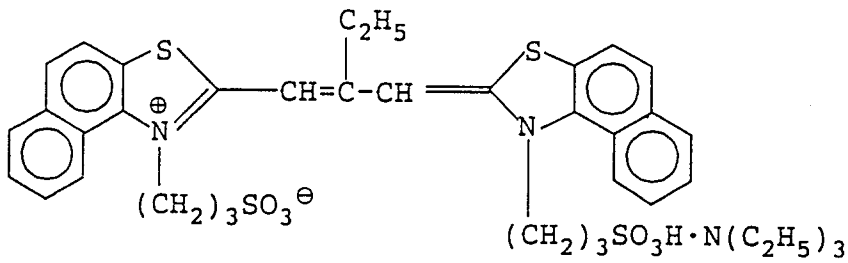

Sensitizing Dye S-2

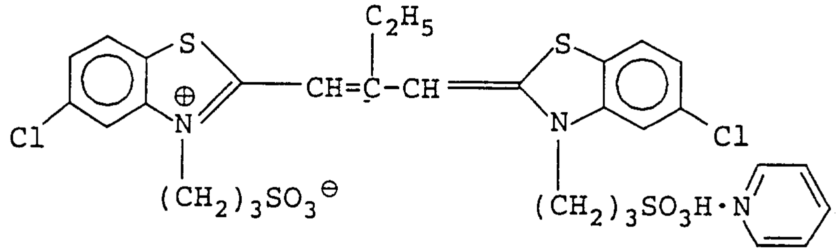

Sensitizing Dye S-3

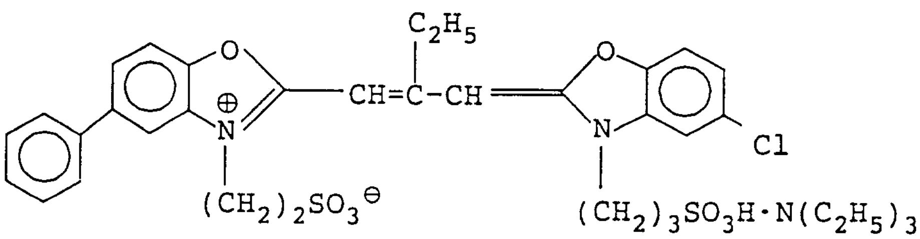

Sensitizing Dye S-4

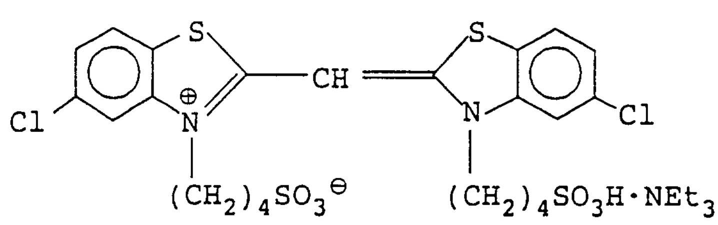

(2) Preparation of the gelatin dispersion of the dye-providing compound and electron-providing material

The method for preparing the dispersion of the cyan dye-providing compound and electron-providing material will be described below.

Ethyl acetate 9.5 ml was added to the Cyan Dye-providing Compound (1) 2.12 g, the Electron-providing Material (1) 0.85 g, the High Boiling Solvent (1) 0.35 g and the Surface Active Agent (1) 0.7 g and heating

was applied at about 60 °C to dissolve them to a homogeneous solution. This solution was mixed with a 14% solution 4.5 g of lime-treated gelatin and water 16 ml and then dispersed with a homogenizer at 10,000 rpm for 10 minutes. The resulting gelatin dispersion was designated as Gelatin Dispersion C-1.

The Gelatin Dispersions C-2, M-1, M-2, Y-1, and Y-2 each shown in Table 1 were prepared in the same manner as Gelatin Dispersion C-1 except that the Cyan Dye-providing Compound (1) was replaced with the cyan dye-providing compounds indicated in Table 1.

TABLE 1

| Gelatin dispersion | Dye-providing compound |
|---|---|
| C-1 | Cyan dye-providing compound (1) |
| C-2 | Cyan dye-providing compound (2)* |
| M-1 | Magenta dye-providing compound (1) |
| M-2 | Magenta dye-providing compound (2)** |
| Y-1 | Yellow dye-providing compound (1) |
| Y-2 | Yellow dye-providing compound (2)*** |

* Compound No. 38 of the present invention
** Compound No. 37 of the present invention
*** Compound No. 39 of the present invention

Cyan dye-providing compound (1)

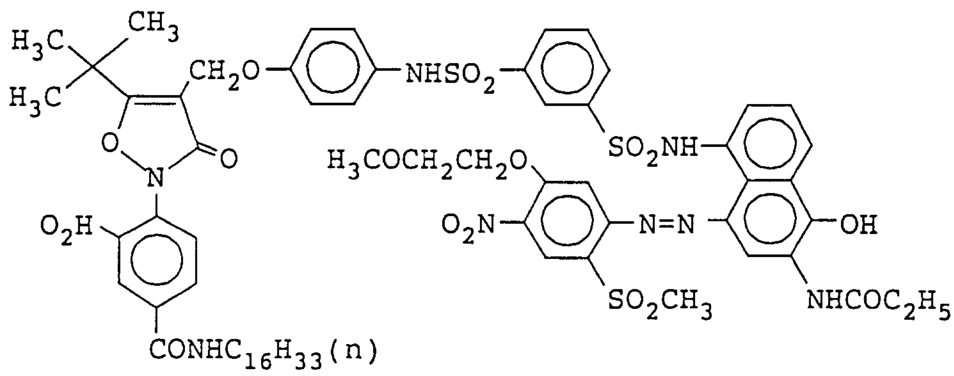

Electron-providing Material (1)

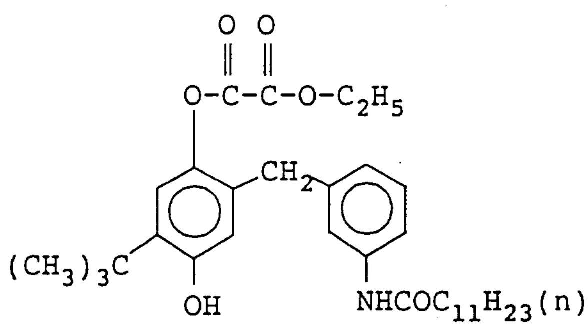

High Boiling Solvent (1)

Tricyclohexyl phosphate

Surface Active Agent (1)

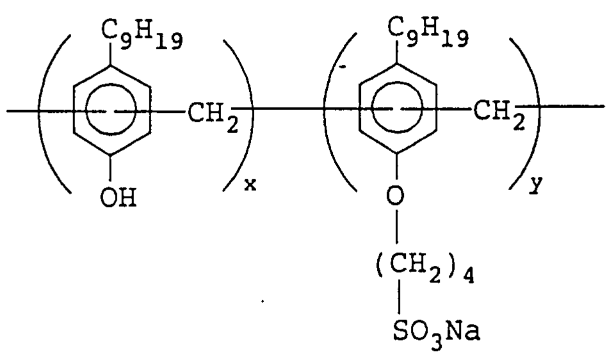

x/y ≒ 58/42 (by moles)

Yellow Dye-providing Compound (1)

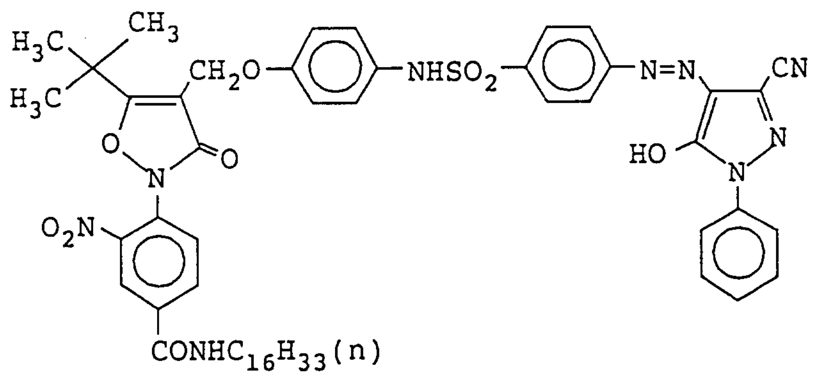

Magenta Dye-providing Compound (1)

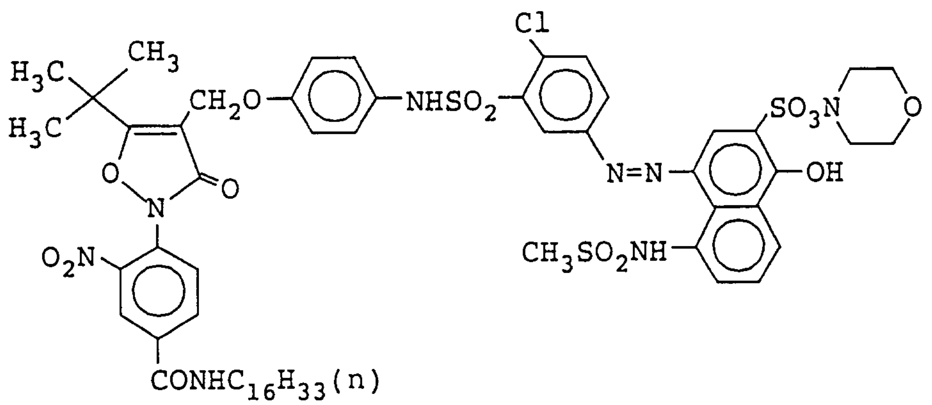

(3) Preparation of the gelatin dispersion of a non-diffusible reducing agent for an intermediate layer:

The method for preparing the dispersion of the non-diffusible reducing agent for the intermediate layer will be described below.

Ethyl acetate 30 ml was added to the Non-diffusible Reducing Agent (1) 23.5 g, the Non-diffusible Reducing Agent (2) 6.9 g, and the High Boiling Solvent (1) 8.5 g and heating was applied at about 60 °C to dissolve them to a homogeneous solution. This solution was mixed with a 10% solution 100 g of lime-treated gelatin, a 5% aqueous solution 15 ml of the Surface Active Agent (2) and water 16 ml and then dispersed with a homogenizer at 10,000 rpm for 10 minutes.

Non-diffusible Reducing Agent (1)

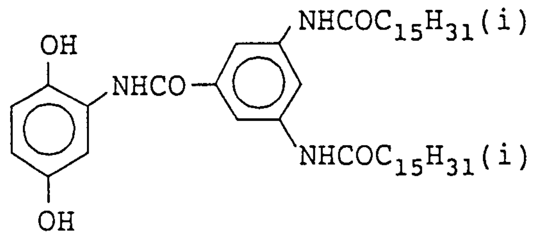

Non-diffusible Reducing Agent (2)

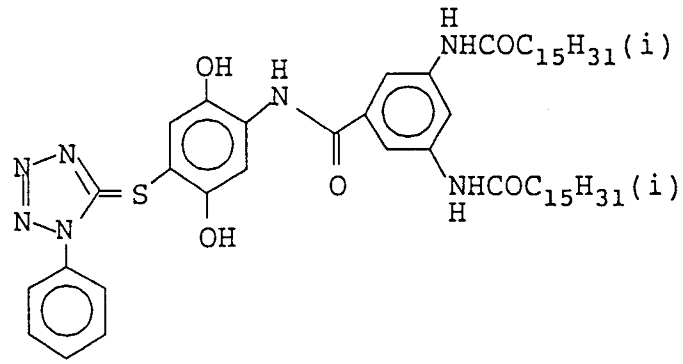

Surface Active Agent (2)

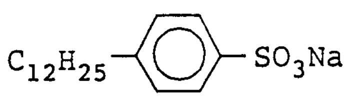

(4) Preparation of the light-sensitive element

C-1, M-1 and Y-1 were used to prepare the Comparative Light-sensitive Element 101 containing the dye-providing compound and light-sensitive silver halide emulsion in different layers and having the construction shown in Tables 2 and 3; C-1, M-1 and Y-1 were used to prepare the Comparative Light-sensitive Element 102 containing the dye-providing compound and light-sensitive silver halide emulsion in the same layer and having the construction shown in Tables 4 and 5; and C-2, M-2 and Y-2 were used to prepare the Comparative Light-sensitive Element 103 containing the dye-providing compound and light-sensitive silver halide emulsion in the same layer and having the construction shown in Tables 6 and 7.

TABLE 2

| Layer No. | Layer name | Additive | Coated amount ($g/m^2$) |
|---|---|---|---|
| 6th layer | Green-sensitive layer | Emulsion for green-sensitive layer | 0.32 (as Ag) |
| | | Gelatin | 0.38 |
| | | Anti-fogging agent | $9.9\times10^{-5}$ |
| | | Surface Active Agent (2) | $6.5\times10^{-3}$ |
| | | Water Soluble Polymer (1) | $6.5\times10^{-3}$ |
| 5th layer | Magenta dye layer | Magenta Dye-providing Compound (1) | 0.39 |
| | | Electron-providing Material (1) | 0.17 |
| | | Gelatin | 0.39 |
| | | High Boiling Solvent (1) | $6.5\times10^{-2}$ |
| | | Surface Active Agent (1) | 0.12 |
| | | Water Soluble Polymer (1) | $8.5\times10^{-3}$ |
| 4th layer | Intermediate layer (2) | Gelatin | 0.32 |
| | | Matting agent (1) | $2.2\times10^{-2}$ |
| | | Surface Active Agent (3) | $1.1\times10^{-2}$ |
| | | Water Soluble Polymer (1) | $1.2\times10^{-2}$ |
| 3rd layer | Intermediate layer (1) | Non-diffusible Reducing Agent (1) | 0.30 |
| | | Non-diffusible Reducing Agent (2) | $8.8\times10^{-2}$ |
| | | Gelatin | 0.69 |
| | | High Boiling Solvent (1) | 0.13 |

| | | | |
|---|---|---|---|
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |
| 2nd layer | Red-sensitive layer | Emulsion for red-sensitive layer | 0.27 (as Ag) |
| | | Gelatin | 0.38 |
| | | Anti-fogging agent (1) | $8.5\times10^{-5}$ |
| | | Surface active agent (2) | $6.5\times10^{-3}$ |
| | | Water soluble polymer (1) | $6.8\times10^{-3}$ |
| 1st layer | Cyan color material layer | Cyan dye-providing compound (1) | 0.41 |
| | | Electron-providing material (1) | 0.16 |
| | | Gelatin | 0.40 |
| | | High boiling solvent (1) | $6.8\times10^{-2}$ |
| | | Surface active agent (1) | 0.14 |
| | | Water soluble polymer (1) | $1.0\times10^{-2}$ |
| Support (polyethylene terephthalate 100 µ) | | | |
| Back layer | | Carbon black | 4.0 |
| | | Gelatin | 2.0 |

TABLE 3

| Layer No. | Layer name | Additive | Coated amount ($g/m^2$) |
|---|---|---|---|
| 12th layer | Protective layer | Gelatin | 0.40 |
| | | Matting Agent (1) | 0.25 |

| | | | |
|---|---|---|---|
| | | Hardener (1) | $4.8\times10^{-2}$ |
| | | Surface Active Agent (1) | $1.2\times10^{-2}$ |
| | | Citric acid | $1.6\times10^{-2}$ |
| | | Water Soluble Polymer (1) | $5.8\times10^{-2}$ |
| 11th layer | UV absorbing layer | Gelatin | 0.47 |
| | | UV Absorber (1) | 0.14 |
| | | UV Absorber (2) | 0.13 |
| | | Surface Active Agent (2) | $7.0\times10^{-3}$ |
| | | Water Soluble Polymer (1) | $7.0\times10^{-2}$ |
| 10th layer | Blue-sensitive layer | Emulsion for blue-sensitive layer | 0.50 (as Ag) |
| | | Gelatin | 0.41 |
| | | Anti-fogging Agent (1) | $1.6\times10^{-4}$ |
| | | Surface Active Agent (2) | $6.5\times10^{-3}$ |
| | | Water Soluble Polymer (1) | $6.8\times10^{-3}$ |
| 9th layer | Yellow color material layer | Yellow Dye-providing Compound (1) | 0.42 |
| | | Electron-providing Material (1) | 0.23 |
| | | Gelatin | 0.51 |
| | | High Boiling Solvent (1) | $6.8\times10^{-2}$ |
| | | Surface Active Agent (2) | 0.14 |
| | | Water Soluble Polymer (1) | $5.1\times10^{-3}$ |
| 8th layer | Inter-mediate layer (2) | Gelatin | 0.32 |
| | | Matting agent (1) | $2.2\times10^{-2}$ |
| | | Surface Active Agent (2) | $1.1\times10^{-2}$ |

| | | | |
|---|---|---|---|
| | | Water Soluble Polymer (1) | $1.2\times10^{-2}$ |
| 7th layer | Inter-mediate layer (3) | Non-diffusible Reducing Agent (1) | 0.30 |
| | | Non-diffusible Reducing Agent (2) | $8.8\times10^{-2}$ |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |

TABLE 4

| Layer No. | Layer name | Additive | Coated amount (g/m$^2$) |
|---|---|---|---|
| 6th layer | Inter-mediate layer | Gelatin | 0.15 |
| | | Surface Active Agent (2) | $3.3\times10^{-3}$ |
| | | Water Soluble Polymer (1) | $6.8\times10^{-3}$ |
| 5th layer | Green-sensitive layer | Emulsion for green-sensitive layer | 0.32 (as Ag) |
| | | Anti-fogging Agent (1) | $9.9\times10^{-5}$ |
| | | Magenta Dye-providing Compound (1) | 0.39 |
| | | Electron-providing Material (1) | 0.17 |
| | | Gelatin | 0.39 |
| | | High Boiling Solvent (1) | $6.5\times10^{-2}$ |
| | | Surface Active Agent (1) | 0.12 |

| | | | |
|---|---|---|---|
| | | Surface Active Agent (2) | $3.2\times10^{-3}$ |
| | | Water Soluble Polymer (1) | $8.5\times10^{-3}$ |
| 4th layer | Inter-mediate layer (3) | Gelatin | 0.32 |
| | | Matting Agent (1) | $2.2\times10^{-2}$ |
| | | Surface Active Agent (3) | $1.1\times10^{-2}$ |
| | | Water Soluble Polymer (1) | $1.2\times10^{-2}$ |
| 3rd layer | Inter-mediate layer (2) | Non-diffusible Reducing Agent (1) | 0.30 |
| | | Non-diffusible reducing Agent (2) | $8.8\times10^{-2}$ |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |
| 2nd layer | Inter-mediate layer (1) | Gelatin | 0.15 |
| | | Surface Active Agent (2) | $3.3\times10^{-3}$ |
| | | Water Soluble Polymer (1) | $6.8\times10^{-3}$ |
| 1st layer | Red-sensitive layer | Emulsion for red-sensitive layer | 0.27 (as Ag) |
| | | Anti-fogging Agent (1) | $8.5\times10^{-5}$ |
| | | Cyan Dye-providing Compound (1) | 0.41 |
| | | Electron-providing Material (1) | 0.16 |
| | | Gelatin | 0.40 |

| | | | |
|---|---|---|---|
| | | High Boiling Solvent (1) | $6.8\times10^{-2}$ |
| | | Surface Active Agent (1) | 0.14 |
| | | Surface Active Agent (2) | $3.2\times10^{-3}$ |
| | | Water Soluble Polymer (1) | $1.0\times10^{-2}$ |
| Support (polyethylene terephthalate 100 μ) | | | |
| Back layer | | Carbon black | 4.0 |
| | | Gelatin | 2.0 |

TABLE 5

| Layer No. | Layer name | Additive | Coated amount ($g/m^2$) |
|---|---|---|---|
| 11th layer | Protective layer | Gelatin | 0.40 |
| | | Matting Agent (1) | 0.25 |
| | | Hardener (1) | $4.8\times10^{-2}$ |
| | | Surface Active Agent (2) | $1.2\times10^{-2}$ |
| | | Citric acid | $1.6\times10^{-2}$ |
| | | Water Soluble Polymer (1) | $5.8\times10^{-2}$ |
| 10th layer | UV absorbing layer | Gelatin | 0.47 |
| | | UV Absorber (1) | 0.14 |
| | | UV Absorber (2) | 0.13 |
| | | Surface Active Agent (2) | $7.0\times10^{-3}$ |
| | | Water Soluble Polymer (1) | $7.0\times10^{-2}$ |
| 9th layer | Blue-sensitive layer | Emulsion for blue-sensitive layer | 0.50 (as Ag) |
| | | Anti-fogging Agent (1) | $1.6\times10^{-4}$ |
| | | Yellow Dye-providing Compound (1) | 0.42 |

| | | | |
|---|---|---|---|
| | | Electron-providing Material (1) | 0.23 |
| | | Gelatin | 0.92 |
| | | High Boiling Solvent (1) | $6.8\times10^{-2}$ |
| | | Surface Active Agent (1) | 0.14 |
| | | Surface Active Agent (2) | $6.5\times10^{-3}$ |
| | | Water Soluble Polymer (1) | $6.8\times10^{-3}$ |
| 8th layer | Inter-mediate layer (6) | Gelatin | 0.32 |
| | | Matting Agent (1) | $2.2\times10^{-2}$ |
| | | Surface Active Agent (2) | $1.1\times10^{-2}$ |
| | | Water Soluble Polymer (1) | $1.2\times10^{-2}$ |
| 7th layer | Inter-mediate layer (5) | Non-diffusible Reducing Agent (1) | 0.30 |
| | | Non-diffusible Reducing Agent (2) | $8.8\times10^{-2}$ |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |

TABLE 6

| Layer No. | Layer name | Additive | Coated amount ($g/m^2$) |
|---|---|---|---|
| 6th layer | Inter-mediate layer (4) | Gelatin | 0.15 |
| | | Surface Active Agent (2) | $3.3\times10^{-3}$ |

| | | | |
|---|---|---|---|
| | | Water Soluble Polymer (1) | $6.8\times10^{-3}$ |
| 5th layer | Green-sensitive layer | Emulsion for green-sensitive layer | 0.32 (as Ag) |
| | | Anti-fogging Agent (1) | $9.9\times10^{-5}$ |
| | | Magenta Dye-providing Compound (2) | 0.39 |
| | | Electron-providing Material (1) | 0.17 |
| | | Gelatin | 0.39 |
| | | High Boiling Solvent (1) | $6.5\times10^{-2}$ |
| | | Surface Active Agent (1) | 0.12 |
| | | Surface Active Agent (2) | $3.2\times10^{-3}$ |
| | | Water Soluble Polymer (1) | $8.5\times10^{-3}$ |
| 4th layer | Intermediate layer (3) | Gelatin | 0.32 |
| | | Matting Agent (1) | $2.2\times10^{-2}$ |
| | | Surface Active Agent (3) | $1.1\times10^{-2}$ |
| | | Water Soluble Polymer (1) | $1.2\times10^{-2}$ |
| 3rd layer | Intermediate layer (2) | Non-diffusible Reducing Agent (1) | 0.30 |
| | | Non-diffusible Reducing Agent (2) | $8.8\times10^{-2}$ |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |

| | | | |
|---|---|---|---|
| 2nd layer | Intermediate layer (1) | Gelatin | 0.15 |
| | | Surface Active Agent (2) | $3.3\times10^{-3}$ |
| | | Water Soluble Polymer (1) | $6.8\times10^{-3}$ |
| 1st layer | Red-sensitive layer | Emulsion for red-sensitive layer | 0.27 (as Ag) |
| | | Anti-fogging Agent (1) | $8.5\times10^{-5}$ |
| | | Cyan Dye-providing Compound (2) | 0.41 |
| | | Electron-providing Material (1) | 0.16 |
| | | Gelatin | 0.40 |
| | | High Boiling Solvent (1) | $6.8\times10^{-2}$ |
| | | Surface Active Agent (1) | 0.14 |
| | | Surface Active Agent (2) | $3.2\times10^{-3}$ |
| | | Water Soluble Polymer (1) | $1.0\times10^{-2}$ |
| Support (polyethylene terephthalate 100 μ) | | | |
| Back layer | | Carbon black | 4.0 |
| | | Gelatin | 2.0 |

TABLE 7

| Layer No. | Layer name | Additive | Coated amount ($g/m^2$) |
|---|---|---|---|
| 11th layer | Protective layer | Gelatin | 0.40 |
| | | Matting Agent (1) | 0.25 |
| | | Hardener (1) | $4.8\times10^{-2}$ |
| | | Surface Active Agent (3) | $1.2\times10^{-2}$ |
| | | Citric acid | $1.6\times10^{-2}$ |

| | | | |
|---|---|---|---|
| | | Water Soluble Polymer (1) | $5.8\times10^{-2}$ |
| 10th layer | UV absorbing layer | Gelatin | 0.47 |
| | | UV Absorber (1) | 0.14 |
| | | UV Absorber (2) | 0.13 |
| | | Surface Active Agent (2) | $7.0\times10^{-3}$ |
| | | Water Soluble Polymer (1) | $7.0\times10^{-2}$ |
| 9th layer | Blue-sensitive layer | Emulsion for blue-sensitive layer | 0.50 (as Ag) |
| | | Anti-fogging Agent (1) | $1.6\times10^{-4}$ |
| | | Yellow Dye-providing Compound (1) | 0.42 |
| | | Electron-providing Material (1) | 0.23 |
| | | Gelatin | 0.92 |
| | | High Boiling Solvent (1) | $6.8\times10^{-2}$ |
| | | Surface Active Agent (1) | 0.14 |
| | | Surface Active Agent (2) | $6.5\times10^{-3}$ |
| | | Water Soluble Polymer (1) | $6.8\times10^{-2}$ |
| 8th layer | Intermediate layer (6) | Gelatin | 0.32 |
| | | Matting Agent (1) | $2.2\times10^{-2}$ |
| | | Surface Active Agent (2) | $1.1\times10^{-2}$ |
| | | Water Soluble Polymer (1) | $1.2\times10^{-2}$ |
| 7th layer | Intermediate layer (5) | Non-diffusible Reducing Agent (1) | 0.30 |
| | | Non-diffusible Reducing Agent (2) | $8.8\times10^{-2}$ |
| | | Gelatin | 0.69 |

| | |
|---|---|
| High Boiling Solvent (1) | 0.13 |
| Surface Active Agent (2) | $1.1\times10^{-3}$ |
| Stabilizer (1) | $2.4\times10^{-3}$ |
| Citric acid | $1.4\times10^{-2}$ |
| Water Soluble Polymer (1) | $2.8\times10^{-2}$ |

Hardener (1)

1,2-Bis(vinylsulfonylacetamide) ethane

Matting Agent (1)

Polymethyl methacrylate spherical latex (average particle size: 4 µm)

UV Absorber (1)

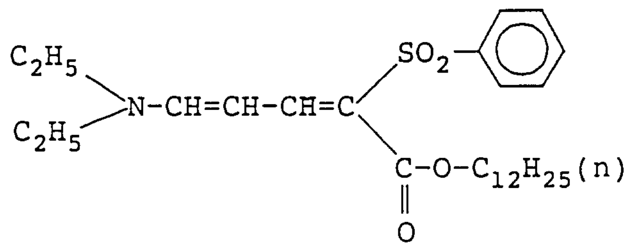

UV Absorber (2)

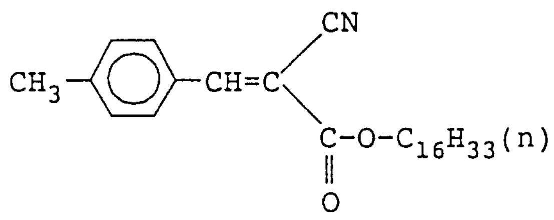

Water Soluble Polymer (1)

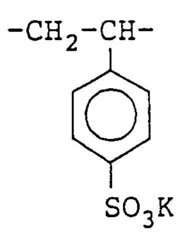

Surface Active Agent (3)

Aerosol OT

Anti-fogging Agent (1)

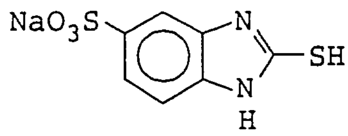

Stabilizer (1)

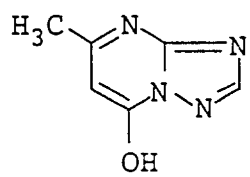

(5) Preparation of the image-receiving element

The image-receiving element was prepared in the following manner.

Paper support: polyethylene 30 µm thick was laminated on both sides of a paper with a thickness of 150 µm. Titanium oxide was added and dispersed in polyethylene provided on the image-receiving layer side in the proportion of 10% by weight based on polyethylene.

Back side:

(a) a light shielding layer containing carbon black 4.0 $g/m^2$ and gelatin 2.0 $g/m^2$.
(b) a white color layer containing titanium oxide 8.0 $g/m^2$ and gelatin 1.0 $g/m^2$.
(c) a protective layer containing gelatin 0.6 $g/m^2$.

The layers (a) to (c) were coated in this order and hardened with a hardener.

Image-receiving layer side:

1) A neutralizing layer containing 22 $g/m^2$ of an acrylic acid-butyl acrylate (mole ratio: 8:2) copolymer having an average molecular weight of 50,000.
2) A second timing layer containing a 4.5 $g/m^2$ total coating in terms of a weight ratio 95 : 5 of cellulose acetate with an acetyl value of 51.3% (the weight of acetic acid released by a hydrolysis was 0.513 g per g of the sample), and a styrene-maleic anhydride (mole ratio: 1:1) copolymer having an average molecular weight of 10,000.
3) An intermediate layer containing poly-2-hydroxyethyl methacrylate 0.4 $g/m^2$.
4) A first timing layer containing 1.6 $g/m^2$ on a solids basis of a polymer latex prepared by emulsion polymerizing styrene/butyl acrylate/acrylic acid/N-methylolacrylamide in a weight ratio 49.7/42.3/4/4, and a polymer latex prepared by emulsion polymerizing methyl methacrylate/acrylic acid/N-methylolacrylamide in a weight ratio 93/33/4, wherein they were blended so that the solid matter ratio thereof became 6:4.
5) An image-receiving layer which was provided by coating a polymer mordant having the following repetitive unit 3.0 $g/m^2$ and gelatin 3.0 $g/m^2$ under the presence of a coating aid.

Coating aid

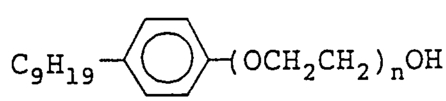

(n = 30)

Repetitive unit

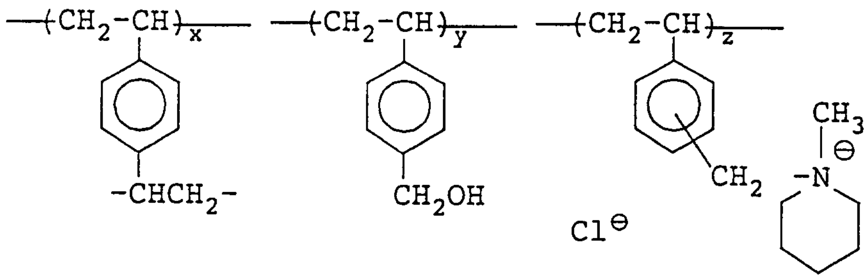

x : y : z = 5 : 5 : 90 (by moles)

6) A protective layer containing gelatin 0.6 g/$m^2$.

The layers (1) through (6) were coated in this order and hardened with a hardener.

(6) Preparation of the processing solution

The preparation of the processing solution is shown below.

A processing solution 0.8 g of the following composition was filled in a breakable vessel:

| | |
|---|---|
| 1-p-Tolyl-4-hydroxymethyl-4-methyl-3-pyrazolidone | 10.0 g |
| 1-Phenyl-4-hydroxymethyl-4-methyl-pyrazolidone | 4.0 g |
| Potassium sulfite.(anhydrous) | 4.0 g |
| Hydroxyethyl cellulose | 40 g |
| Potassium hydroxide | 64 g |
| Benzyl alcohol | 2.0 g |
| Water to make | 1 kg |

(7) Experiment results

The above Light-sensitive Elements 101 to 103 were exposed from the emulsion layer side via a color separation filter of grey, R, G and B and then the above processing solution was spread between the light-sensitive element and the image-receiving layer of the image-receiving element and superposed thereon with the aid of a pressurized roller so that the thickness thereof became 60 μm. The processing was carried out at 25 °C and 1.5 minutes later the light-sensitive element and image-receiving element were separated.

There were measured with a color densitometer, the maximum densities (Dmax) and minimum densities (Dmin) of cyan, magenta and yellow at the grey portions transferred on the respective image-receiving elements, the sensitivities, and the color reproducibilities at the color separation portions.

The results are shown in Table-8.

TABLE 8

| | | Sample No. | | |
|---|---|---|---|---|
| | | 101 (Comp.) | 102 (Comp.) | 103 (Inv.) |
| Dmax | Cyan<br>Magenta<br>Yellow | 2.25<br>2.30<br>2.00 | 2.20<br>2.30<br>1.95 | 2.25<br>2.31<br>2.08 |
| Dmin | Cyan<br>Magenta<br>Yellow | 0.19<br>0.20<br>0.18 | 0.15<br>0.16<br>0.12 | 0.14<br>0.14<br>0.11 |
| Sensitivity* | Cyan<br>Magenta<br>Yellow | 100<br>100<br>100 | 40<br>36<br>45 | 95<br>92<br>92 |
| Red light portion | Magenta<br>Yellow | 1.60<br>1.85 | 2.00<br>1.90 | 2.19<br>1.90 |
| Green light portion | Cyan<br>Yellow | 1.95<br>1.60 | 2.15<br>1.90 | 2.20<br>1.90 |
| Blue light portion | Magenta<br>Cyan | 1.85<br>2.00 | 2.15<br>2.20 | 2.15<br>2.20 |

* Relative sensitivity = a relative value (anti-logarithm), where the sensitivity at a density of 1.0 in each layer of the Light-sensitive Element 101 was taken as 100.

It can be seen from the results summarized in Table 8 that the Light-sensitive Elements 102 and 103 in which the light-sensitive silver halide emulsions and dye-providing compounds were incorporated into the same layer have lowered densities at the minimum density portions and in addition, improved color reproducibilities compared with those of the Light-sensitive Element 101 in which they were incorporated into different layers. Further, it can be seen that the Light-sensitive Element 103 of the present invention was improved to a large extent compared with the Light-sensitive Element 102 and it is apparent that the present invention does not cause deterioration of the sensitivity and is advantageous for improving the S/N ratio of an image and the color reproducibility.

EXAMPLE 2

The Light-sensitive Elements 104 shown in Table 9, 105 shown in Table 10 and 106 shown in Table 11 were prepared in the same manner as Example 1 except that the Gelatin Dispersions M-1 and M-2 were replaced with Gelatin Dispersion M-3 in which the dye-providing compound was replaced with the following Magenta Dye-providing Compound (3).

TABLE 9

| Layer No. | Layer name | Additive | Coated amount ($g/m^2$) |
|---|---|---|---|
| 4th layer | Protective layer | Gelatin | 0.40 |
| | | Matting Agent (1) | 0.25 |
| | | Hardener (1) | $1.6 \times 10^{-2}$ |
| | | Surface Active Agent (1) | $1.2 \times 10^{-2}$ |
| | | Citric acid | $1.6 \times 10^{-2}$ |
| | | Water Soluble Polymer (1) | $5.8 \times 10^{-2}$ |
| 3rd layer | Inter-mediate layer | Non-diffusible Reducing Agent (1) | 0.30 |
| | | Gelatin | 0.69 |
| | | High Boiling Solvent (1) | 0.13 |
| | | Surface Active Agent (2) | $1.1 \times 10^{-3}$ |
| | | Stabilizer (1) | $2.4 \times 10^{-3}$ |
| | | Citric acid | $1.4 \times 10^{-2}$ |
| | | Water Soluble Polymer (1) | $2.8 \times 10^{-2}$ |
| 2nd layer | Green-sensitive layer | Emulsion for green-sensitive layer | 0.32 (as Ag) |
| | | Gelatin | 3.38 |
| | | Anti-fogging Agent (1) | $9.9 \times 10^{-5}$ |
| | | Surface Active Agent (2) | $6.5 \times 10^{-3}$ |
| | | Water Soluble Polymer (1) | $6.8 \times 10^{-3}$ |
| 1st layer | Magenta color material layer | Magenta Dye-providing Compound (1) | 0.39 |
| | | Electron-providing Material (1) | 0.17 |

| | | | |
|---|---|---|---|
| | | Gelatin | 0.39 |
| | | High Boiling Solvent (1) | $6.5\times10^{-2}$ |
| | | Surface Active Agent (1) | 0.12 |
| | | Water Soluble Polymer (1) | $8.5\times10^{-3}$ |
| Support (polyethylene terephthalate 100 μ) | | | |
| Back layer | | Carbon black | 4.0 |
| | | Gelatin | 2.0 |

TABLE 10

| Layer No. | Layer name | Additive | Coated amount (g/m$^2$) |
|---|---|---|---|
| 4th layer | Protective layer | Gelatin | 0.40 |
| | | Matting Agent (1) | 0.25 |
| | | Hardener (1) | $1.6\times10^{-2}$ |
| | | Surface Active Agent (3) | $1.2\times10^{-2}$ |
| | | Citric acid | $1.6\times10^{-2}$ |
| | | Water Soluble Polymer (1) | $5.8\times10^{-2}$ |
| 3rd layer | Inter-mediate layer (1) | Non-diffusible Reducing Agent (1) | 0.30 |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |

| | | | |
|---|---|---|---|
| 2nd layer | Intermediate layer (2) | Gelatin | 0.15 |
| | | Surface Active Agent (2) | $3.3\times10^{-3}$ |
| | | Water Soluble Polymer (1) | $6.8\times10^{-3}$ |
| 1st layer | Green-sensitive layer | Emulsion for green-sensitive layer | 0.32 (as Ag) |
| | | Anti-fogging Agent (1) | $9.9\times10^{-5}$ |
| | | Magenta Dye-providing Compound (2) | 0.39 |
| | | Electron-providing Material (1) | 0.17 |
| | | Gelatin | 0.39 |
| | | High Boiling Solvent (1) | $6.5\times10^{-2}$ |
| | | Surface Active Agent (1) | 0.12 |
| | | Surface Active Agent (2) | $3.2\times10^{-3}$ |
| | | Water Soluble Polymer (1) | $8.5\times10^{-3}$ |
| Support (polyethylene terephthalate 100 µ) | | | |
| Back layer | | Carbon black | 4.0 |
| | | Gelatin | 2.0 |

TABLE 11

| Layer No. | Layer name | Additive | Coated amount ($g/m^2$) |
|---|---|---|---|
| 4th layer | Protective layer | Gelatin | 0.40 |
| | | Matting Agent (1) | 0.25 |
| | | Hardener (1) | $1.6\times10^{-2}$ |
| | | Surface Active Agent (3) | $1.2\times10^{-2}$ |
| | | Citric acid | $1.6\times10^{-2}$ |

| | | | |
|---|---|---|---|
| | | Water Soluble Polymer (1) | $5.8\times10^{-2}$ |
| 3rd layer | Intermediate layer (1) | Non-diffusible Reducing Agent (1) | 0.30 |
| | | Gelatin | 0.69 |
| | | High Boiling Solvent (1) | 0.13 |
| | | Surface Active Agent (2) | $1.1\times10^{-3}$ |
| | | Stabilizer (1) | $2.4\times10^{-3}$ |
| | | Citric acid | $1.4\times10^{-2}$ |
| | | Water Soluble Polymer (1) | $2.8\times10^{-2}$ |
| 2nd layer | Intermediate layer (2) | Gelatin | 0.15 |
| | | Surface Active Agent (2) | $3.3\times10^{-3}$ |
| | | Water Soluble Polymer (1) | $6.8\times10^{-3}$ |
| 1st layer | Green-sensitive layer | Emulsion for green-sensitive layer | 0.32 (as Ag) |
| | | Anti-fogging Agent (1) | $9.9\times10^{-5}$ |
| | | Magenta Dye-providing compound (3) | 0.39 |
| | | Electron-providing Material (1) | 0.17 |
| | | Gelatin | 0.39 |
| | | High Boiling Solvent (1) | $6.5\times10^{-2}$ |
| | | Surface Active Agent (1) | 0.12 |
| | | Surface Active Agent (2) | $3.2\times10^{-3}$ |
| | | Water Soluble Polymer (1) | $8.5\times10^{-3}$ |
| Support (polyethylene terephthalate 100 μ) | | | |
| Back layer | | Carbon black | 4.0 |
| | | Gelatin | 2.0 |

Magenta dye-providing compound (3)

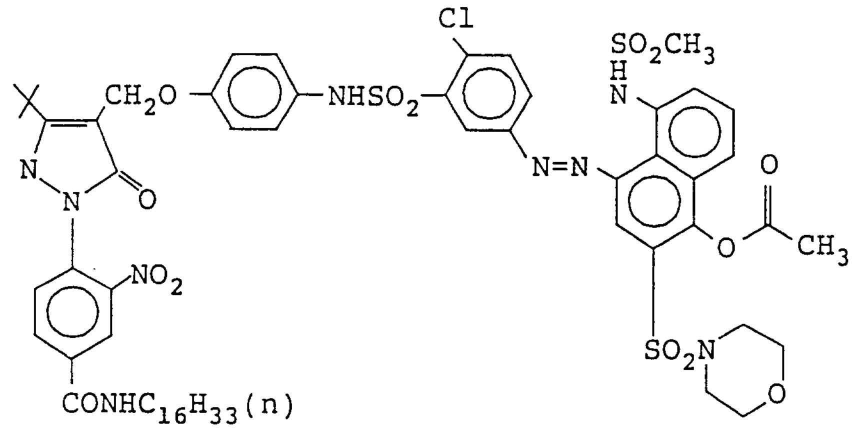

The Light-sensitive Elements 104, 105 and 106 were stored at 45 °C and 80% humidity for 7 days and then were exposed, followed by carrying out the processing and observing the change of the sensitivities before and after storage.

The results are shown in Table 12.

TABLE 12

| | 104 (Comp.) | 105 (Inv.) | 106 (Comp.) |
|---|---|---|---|
| Sensitivity before storage* | 100 | 92 | 92 |
| Sensitivity after storage* | 100 | 92 | 68 |

* Relative sensitivity = a relative value (anti-logarithm), where the sensitivity at a density of 1.0 in the Light-sensitive Element 104 was taken as 100.

It can be seen from the results shown in Table 12 together with the results in Example 1 that the compound of the present invention is very excellent in the storing stability of the temporary shorter wavelength-shifting effect and apparently satisfies the storage stability and rapid hue recoverability in a processing.

## EXAMPLE 3

The Light-sensitive Elements 105 and 106 prepared in Example 2 but not stored and the Light-sensitive Elements 105 and 106 stored in the same condition as that in Example 2 prepared as in Example 2 and then were subjected to removal of the back layers and then to measurement of the respective densities at a wavelength of 550 nm. The density change from that before storage was measured.

The results are shown in Table 13.

TABLE 13

| | 105 (Inv.) | 106 (Comp.) |
|---|---|---|
| Density change after storage | 0.00 | 0.11 |

It was optically substantiated from the results shown in Table 13 that while the magenta density was increased during storage in the Comparative Light-sensitive Element 106, it was not changed in the Light-sensitive Element 105 of the present invention and the compound of the present invention had excellent stability.

EXAMPLE 4

The Dye-providing Compound (No. 37) of the present invention 4.83 mg and the Reducing Agent (A) 36.3 mg were dissolved in tetrahydrofuran 20 ml and maintained at the temperature of 25°C. A buffer solution 20 ml of pH 12.0 maintained similarly at 25°C was added and there was observed the change in the Dye-providing Compound No. (37) and the Dye (B) formed, which was caused according to the progress of the reaction with the addition point set as the starting point.

Reducing agent (A)

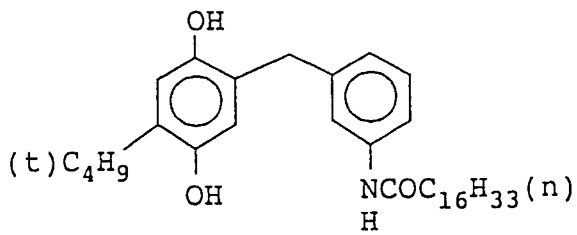

Dye (B)

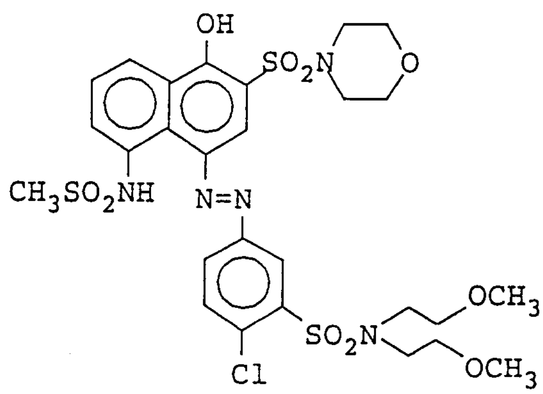

The results are shown in Table 14.

TABLE 14

| | Time (seconds) | | | | |
|---|---|---|---|---|---|
| | 10 | 30 | 60 | 120 | 300 |
| Existing rate of dye (B)(%) | 7 | 20 | 50 | 85 | 100 |

It is apparent from the results shown in Table 14 that the dye-providing compound of the present invention can very rapidly recover its hue by an inter-molecular nucleophilic reaction following a redox reaction with a reducing agent.

EXAMPLE 5

The light-sensitive element having the construction shown in Table 15 was prepared and designated as the Light-sensitive Element 501. Further, the dye fixing element having the construction shown in Table 16 was prepared and designated as the Dye Fixing Element (11). Note that the Fluorescent Whitening Agent

(11), the Anti-stain Agent (11) and the High Boiling Solvent (11) each shown in Table 17 were added in the form of an emulsion prepared by emulsifying them in the presence of the Surface Active Agent (14).

TABLE 15

(Construction of light-sensitive element)

| Layer No. | Layer name | Additive | Coated amount ($mg/m^2$) |
|---|---|---|---|
| 6th layer | Protective layer | Gelatin | 900 |
| | | Silica (size: 4 μm) | 40 |
| | | Zinc hydroxide | 600 |
| | | Surface Active Agent (11) | 130 |
| 5th layer | Blue-sensitive emulsion layer | Emulsion for blue-sensitive emulsion | 380 (as Ag) |
| | | Yellow Dye-providing Compound (11) | 400 |
| | | Gelatin | 600 |
| | | Electron-providing Material (11) | 303 |
| | | High Boiling Solvent (11) | 200 |
| | | Electron Transfer Agent Precursor (11) | 15 |
| | | Zinc hydroxide | 330 |
| | | Anti-fogging Agent (11) | 0.6 |
| 4th layer | Intermediate layer | Gelatin | 700 |

| | | | |
|---|---|---|---|
| | | Electron Transfer Agent Precursor (12) | 130 |
| | | High Boiling Solvent (11) | 48 |
| | | Surface Active Agent (12) | 61 |
| | | Electron Transfer Agent (11) | 27 |
| | | Electron Transfer Agent (12) | 36 |
| | | Hardener (11) | 37 |
| 3rd layer | Green-sensitive layer | Green-sensitive silver halide emulsion | 220 (as Ag) |
| | | Magenta Dye-providing Compound (12) | 365 |
| | | Gelatin | 310 |
| | | Electron-providing Material (11) | 158 |
| | | High Boiling Solvent (11) | 183 |
| | | Electron Transfer Agent (11) | 27 |
| | | Electron Transfer Agent Precursor (11) | 15 |
| | | Anti-fogging agent (12) | 0.3 |
| 2nd layer | Intermediate layer | Gelatin | 790 |
| | | Zinc hydroxide | 300 |
| | | Electron Transfer Agent (12) | 130 |
| | | High Boiling Solvent (11) | 73 |
| | | Surface Active Agent (12) | 100 |
| | | Activated carbon | 25 |
| 1st layer | Red-sensitive layer | Red-sensitive silver halide emulsion | 230 (as Ag) |
| | | Cyan Dye-providing Compound (13) | 343 |

| | |
|---|---|
| Gelatin | 330 |
| Electron-providing Material (11) | 163 |
| High Boiling Solvent (11) | 172 |
| Electron Transfer Agent (11) | 28 |
| Electron Transfer Agent Precursor (11) | 17 |
| Anti-fogging Agent (13) | 0.7 |

Support Polyethylene terephthalate 96 μm

(carbon black was contained in a back layer)

Surface Active Agent (11)

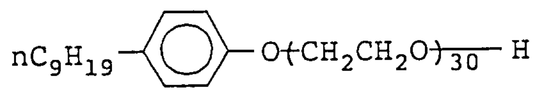

Anti-fogging Agent (11)

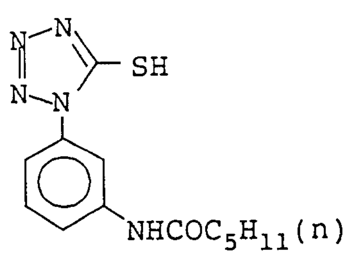

Electron-providing Agent (11)

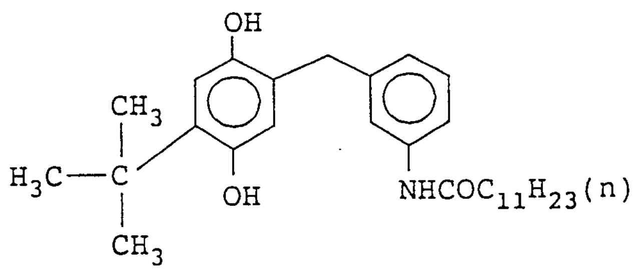

High Boiling Solvent (11)

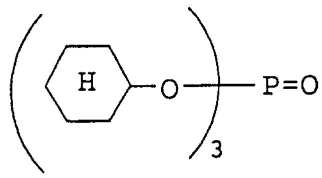

Electron Transfer Agent Precursor (11)

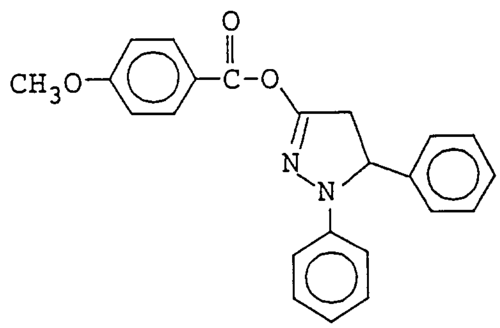

Surface Active Agent (12)

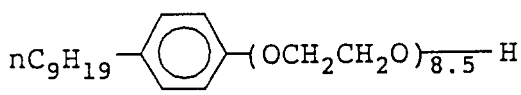

Electron Transfer Agent (11)

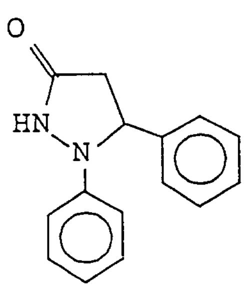

Electron Transfer Agent (12)

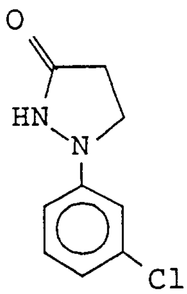

Hardener

$CH_2 = CHSO_2CH_2CONH(CH_2)_2NHCOCH_2SO_2CH = CH_2$ (11)-1

$CH_2 = CHSO_2CH_2CONH(CH_2)_3NHCOCH_2SO_2CH = CH_2$ (11)-2

Anti-fogging Agent (12)

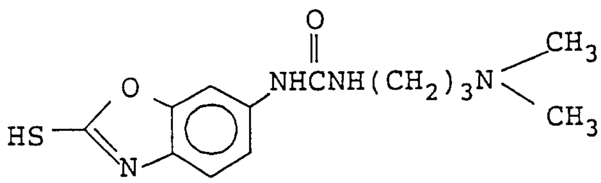

Anti-fogging Agent (13)

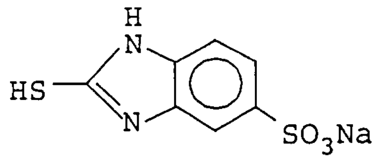

Dye-providing Compound (11)

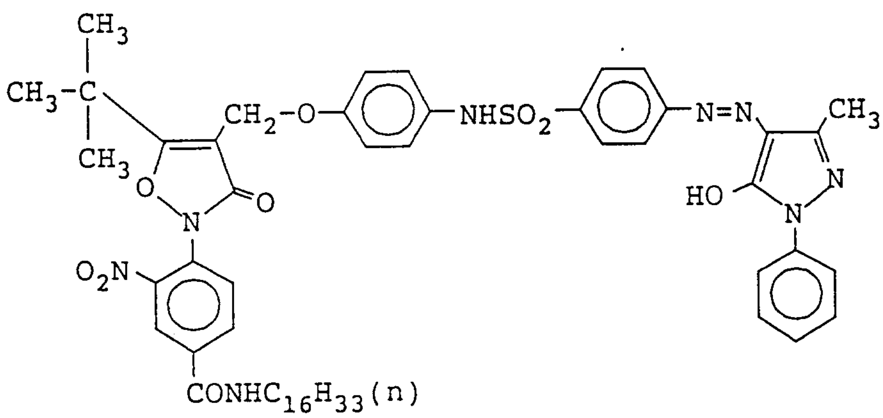

Dye-providing Compound (12)

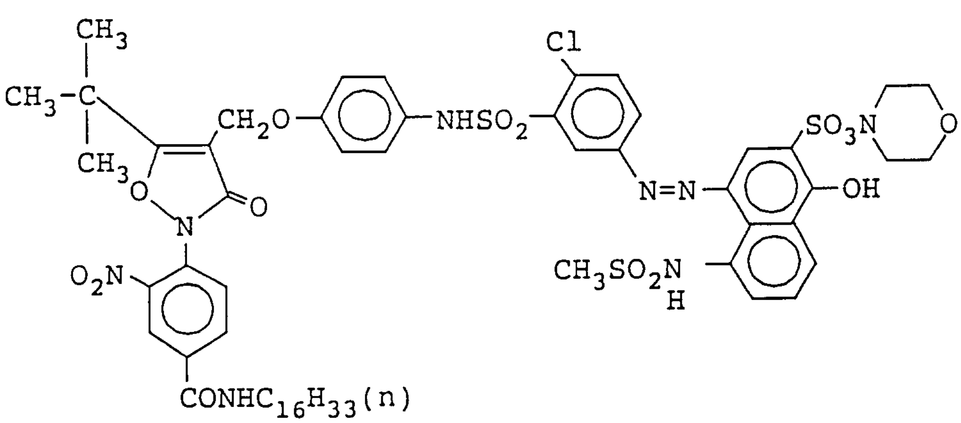

Dye-providing Compound (13)

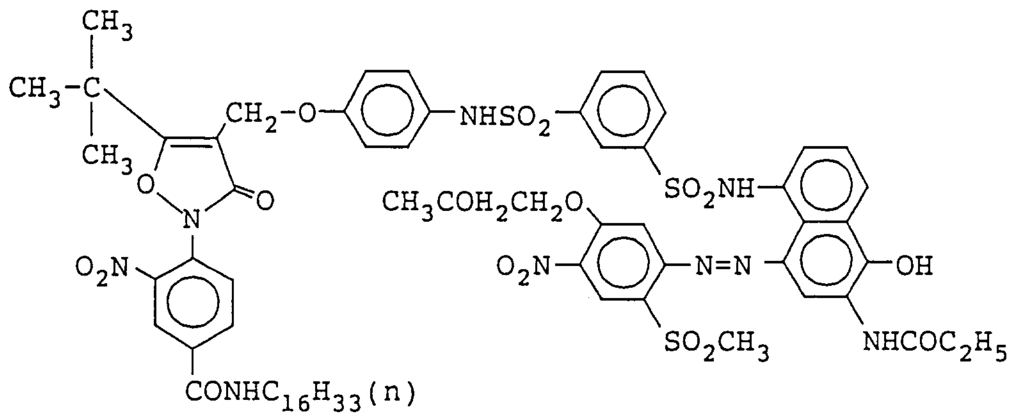

Electron-providing Material (12)

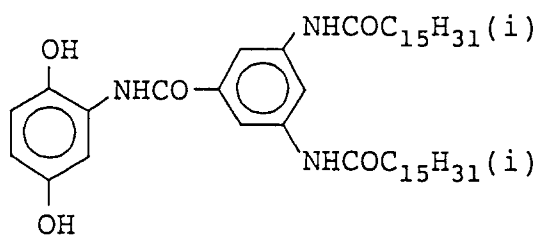

TABLE 16

Structure of the Dye Fixing Element (11)

| |
|---|
| Surface Layer (1) of the layer structure shown in Table 17 |
| Support (1) of the structure shown in Table 19 |
| Back Layer (1) of the layer structure shown in Table 18 |

TABLE 17

Structure of the Surface Layer (1)

| Layer No. | Additive | Coated amount ($g/m^2$) |
| --- | --- | --- |
| 4th layer | Water Soluble Polymer (11) | 0.25 |
| | Water Soluble Polymer (12) | 0.07 |
| | Guanidine picolinate | 0.45 |
| | Surface Active Agent (11) | 0.01 |
| | Surface Active Agent (12) | 0.10 |
| | Surface Active Agent (13) | 0.03 |
| 3rd layer | Gelatin | 0.25 |
| | Water Soluble Polymer (11) | 0.02 |
| | Surface Active Agent (11) | 0.005 |
| | Surface Active Agent (12) | 0.005 |
| | Hardener (11) | 0.16 |
| 2nd layer | Gelatin | 1.40 |
| | Water Soluble Polymer (11) | 0.20 |
| | Water Soluble Polymer (13) | 0.60 |
| | Mordant (11) | 2.40 |
| | Guanidine picolinate | 2.20 |
| | Fluorescent Whitening Agent (11) | 0.055 |
| | Anti-stain Agent (11) | 0.060 |
| | High Boiling Solvent (11) | 1.40 |
| | Surface Active Agent (14) | 0.025 |
| 1st layer | Gelatin | 0.25 |

| | |
|---|---|
| Water Soluble Polymer (11) | 0.02 |
| Surface Active Agent (11) | 0.005 |
| Surface Active Agent (12) | 0.005 |
| Hardener (11) | 0.16 |

TABLE 18

| Structure of the Back Layer (1) | | |
|---|---|---|
| Layer No. | Additive | Coated amount ($g/m^2$) |
| First back layer | Gelatin<br>Water Soluble Polymer (14)<br>Surface Active Agent (11)<br>Hardener (11) | 3.00<br>0.04<br>0.05<br>0.13 |
| Second back layer | Gelatin<br>Water Soluble Polymer (14)<br>Surface Active Agent (11)<br>Surface Active Agent (15)<br>Hardener (11) | 0.37<br>0.005<br>0.045<br>0.011<br>0.03 |

TABLE 19

| Structure of the Support (1) | | |
|---|---|---|
| Layer Name | Composition | Layer thickness (μm) |
| Surface subbing layer | Gelatin | 0.1 |
| Surface PE layer (glossy) | Low density polyethylene (density: 0.923): 89.2 parts<br>Surface-treated titanium oxide: 10.0 parts<br>Ultramarine: 0.8 part | 20.0 |
| Pulp layer<br>Back PE layer (mat) | Wood free paper (LBKP/NBKP = 1:1, density: 1.080)<br>High density polyethylene (density: 0.960) | 73.0<br>18.0 |
| Back subbing layer | Gelatin<br>Colloidal silica | 0.05<br>0.05 |
| | | Total 111.2 |

TABLE 20

| Physical properties of the Support (1) | | | |
|---|---|---|---|
| Item | Unit | Physical property | Measuring method |
| Rigidity (vertical/horizontal)<br>Whiteness degree | g | 4.40/3.15<br>L* 94.20<br>a* +0.12<br>b* -2.75 | Taper rigidity meter<br>CLE L*a*b* |

Water Soluble Polymer (11):

Sumikagel L5-H (manufactured by Sumitomo Chemical Ind. Co.)

Water Soluble Polymer (12):

κ-Carrageenan (manufactured by Taito Co.)

Water Soluble Polymer (13):

Dextran (molecular weight: 70,000)

Water Soluble Polymer (14)

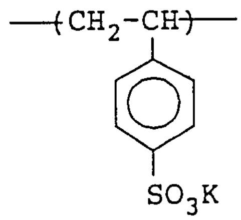

Surface Active Agent (11)

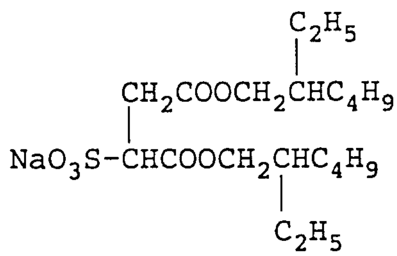

Surface Active Agent (12)

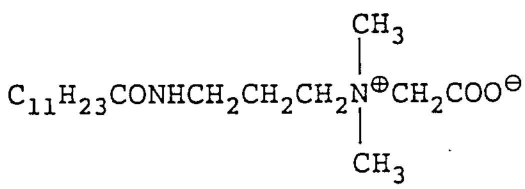

Surface Active Agent (13)

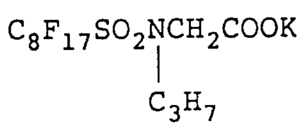

Surface Active Agent (14)

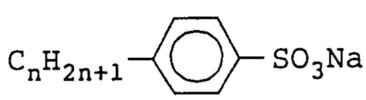

($n \fallingdotseq 12.6$)

Surface Active Agent (15)

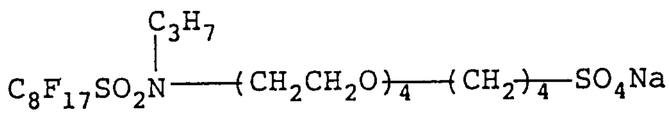

Hardener (11)

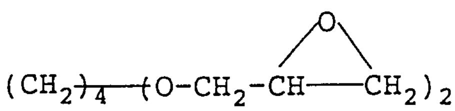

Fluorescent Whitening Agent (11):

2,5-Bis(5-t-benzoxazole) thiophene

High Boiling Solvent (11):

Enpara 40 (manufactured by Ajinomoto Co.)

Matting Agent (11):

Benzoguanamine resin (average grain size: 15 μm)

Mordant (11)

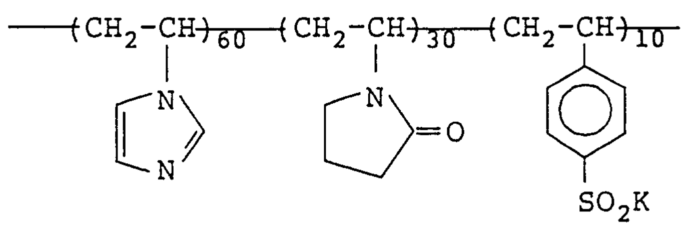

Anti-stain Agent (11)

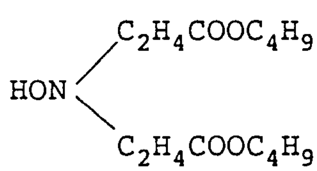

The Light-sensitive Element 502 was prepared in the same manner as above except that the magenta dye-providing compound was replaced with the Compound No. 37 of the present invention and the cyan dye-providing compound was replaced with the Compound No. 38 of the present invention. Further, the Light-sensitive Element 503 was prepared in the same manner except that the magenta dye-providing compound was replaced with the Compound No. 16 of the present invention and the cyan dye-providing compound was replaced with the Compound No. 1 of the present invention.

The above light-sensitive elements and dye fixing elements were processed with the equipment described in JP-A-2-84634.

That is, an original picture (a test chart on which wedges of yellow, magenta, cyan and grey each having a density continuously changing were recorded) was subjected to a scanning exposure on the light-sensitive element through a slit. After this light-sensitive element thus exposed was dipped in water maintained at 40 °C for about 5 seconds, it was squeezed with rollers and immediately superposed on the dye fixing element so that the layer faces thereof were contacted, followed by heating for 15 seconds with a heat roller which was adjusted so that the temperature of the wet faces absorbing water became 80 °C. Then, the light-sensitive element was peeled off from the dye fixing element, whereby a sharp color image corresponding to the original picture was obtained on the dye fixing element.

There were measured the maximum densities (Dmax) and minimum densities (Dmin) of cyan, magenta and yellow at the grey portion transferred on each of the dye fixing elements, and the sensitivities.

The results are shown in Table 21.

TABLE 21

| | | Sample No. | | |
|---|---|---|---|---|
| | | 501 (Comp.) | 502 (Inv.) | 503 (Inv.) |
| Dmax | Cyan | 2.10 | 2.10 | 2.12 |
| | Magenta | 2.25 | 2.22 | 2.23 |
| | Yellow | 2.00 | 1.99 | 1.98 |
| Dmin | Cyan | 0.18 | 0.15 | 0.16 |
| | Magenta | 0.18 | 0.16 | 0.17 |
| | Yellow | 0.14 | 0.14 | 0.14 |
| Sensitivity* | Cyan | 100 | 210 | 205 |
| | Magenta | 100 | 210 | 215 |
| | Yellow | 100 | 100 | 100 |

* Relative sensitivity = a relative value (anti-logarithm), where the sensitivity at a density of 1.0 in each layer of the Light-sensitive Element 501 was taken as 100.

It can be seen from the results shown in Table 21 that the use of a compound of the present invention for the heat developing light-sensitive material provides an excellent image and improves sensitivity. In the Light-sensitive Elements 502 and 503, in which a yellow filter dye was not used, a non-temporarily shorter wavelength-shifted compound was used as the yellow dye-providing compound.

While the invention has been described in detail and with reference to specific embodiments thereof, it will be apparent to one skilled in the art that various changes and modifications can be made therein without departing from the spirit and scope thereof.

## Claims

**1.** A diffusion transfer type silver halide photographic light-sensitive material containing a dye-providing compound represented by the following Formula (I):

REDOX-NuP-E-G-Dye' (I)

wherein G represents an auxochrome of a dye; Dye' represents a group of atoms which remains after the auxochrome is removed from the dye; -G-Dye' represents a group of atoms which is converted to a diffusible dye by cleavage of the bond between G and E; REDOX represents an oxidative group or a reductive group which allows the bond between REDOX and NuP to be cleaved by an oxidation-reduction reaction; NuP represents a nucleophilic group which is released by a bond cleavage reaction between REDOX and NuP triggered by the oxidation-reduction reaction; E represents an electrophilic group which will react with the nucleophilic group NuP and cause the bond between G and E to be cleaved; and REDOX-NuP-E provides the compound represented by Formula (I) with a diffusibility.

2. The diffusion transfer type silver halide photographic light-sensitive material of claim 1, wherein the compound represented by Formula (I) is a reducible dye-providing compound represented by the following Formula (II):

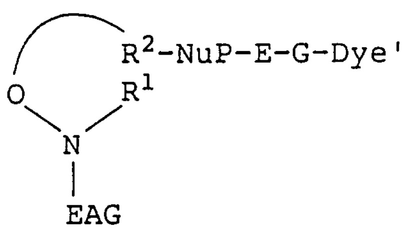

(II)

wherein EAG represents a reducible electron-accepting group; $R^2$ represents a group of atoms which cleaves the bond between $R^2$-NuP when triggered by the cleavage of a nitrogen-oxygen single bond; $R^1$ represents a substituent other than a hydrogen atom; $R^1$ and $R^2$ may be combined with each other to form a ring; and NuP, E, G and Dye' have the same meanings as in Formula (I).

European Patent Office

# EUROPEAN SEARCH REPORT

Application Number

EP 93 10 2790

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | EP-A-0 220 746 (FUJI PHOTO FILM)<br>* page 14, line 40 - page 15, line 45 *<br>* page 23, line 35 - line 41 *<br>* page 69, line 35 - line 50; figure 64 *<br>* page 77, line 14 - line 28 *<br>--- | 1,2 | G03C8/10<br>C07D413/12 |
| Y | US-A-4 564 577 (W.J.BEGLEY)<br>* column 4, line 11 - line 18; claims 1,12; tables 1,3 *<br>--- | 1,2 | |
| A | EP-A-0 198 438 (FUJI PHOTO FILM)<br>* page 23 - page 24 *<br>* page 60, column 115, line 17 - column 116, line 24 *<br>* page 117; figures I-98 *<br>----- | 1,2 | |
| | | | TECHNICAL FIELDS SEARCHED (Int. Cl.5 ) |
| | | | G03C |

The present search report has been drawn up for all claims

| Place of search | Date of completion of the search | Examiner |
|---|---|---|
| THE HAGUE | 07 JUNE 1993 | PHILOSOPH L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)